(19) Europäisches Patentamt
European Patent Office
Office européen des brevets

(11) Publication number: **0 533 933 A1**

(12) **EUROPEAN PATENT APPLICATION published in accordance with Art. 158(3) EPC**

(21) Application number: **91912078.2**

(22) Date of filing: **01.07.91**

(86) International application number: **PCT/JP91/00887**

(87) International publication number: **WO 92/00967 (23.01.92 92/03)**

(51) Int. Cl.5: **C07D 233/76**, C07D 233/84, C07D 233/88, C07D 257/04, C07D 263/44, C07D 263/46, C07D 263/48, C07D 277/34, C07D 277/36, C07D 277/40, C07D 291/04

(30) Priority: **03.07.90 JP 176550/90**
**22.02.91 JP 114184/91**

(43) Date of publication of application: **31.03.93 Bulletin 93/13**

(84) Designated Contracting States: **AT BE CH DE DK ES FR GB GR IT LI LU NL SE**

(71) Applicant: **YAMANOUCHI PHARMACEUTICAL CO. LTD.**
**No. 3-11 Nihonbashi-Honcho, 2-chome**
**Chuo-ku**
**Tokyo103(JP)**

(72) Inventor: **NIIGATA, Kunihiro, 287, Nakabun**
**2-chome**
**Ageo-shi**
**Saitama 362(JP)**
Inventor: **TAKAHASHI, Takumi, 509, Ninomiya**
**2-chome**
**Tsukuba-si**
**Ibaraki 305(JP)**
Inventor: **IWAOKA, Kiyoshi, 5-9, Ninomiya**
**2-chome**
**Tsukuba-shi**
**Ibaraki 305(JP)**
Inventor: **YONEDA, Takashi, 22-7, Namiki**
**3-chome**
**Tsukuba-shi**
**Ibaraki 305(JP)**
Inventor: **NOSHIRO, Osamu, 2240-79,**
**Wakashibacho**
**Ryugasaki-shi**
**Ibaraki 301(JP)**
Inventor: **KOIKE, Reiko, 1456, Ohaza**
**Shimoinayoshi**
**Chiyodamuri**
**Niihari-gun Ibaraki 315(JP)**

(74) Representative: **Geering, Keith Edwin**
**REDDIE & GROSE 16 Theobalds Road**
**London WC1X 8PL (GB)**

(54) **BISHETEROCYCLIC COMPOUND.**

(57) A novel bisheterocyclic compound represented by general formula (I), which is useful as a hypoglycemic drug, and a novel hypoglycemic drug containing the same, wherein $R^1$ and $R^2$ are the same or different from each other and each represents (a), (b), (c), (d) or (e); $R^3$ represents hydrogen, acetylamino or lower alkyl which may be substituted with carboxyl or sulfoxy; X and Y are the same or different from each other and each represents oxygen, sulfur or NH; $B^1$ and $B^2$ represent a benzene or naphthalene ring which may be substituted with lower alkyl; $L^1$ and $L^2$ are the same or different from each other and each represents a single bond, oxygen, sulfur, sulfinyl or sulfonyl; A represents a single bond, linear or branched alkylene, $C_2$ to $C_6$ lower alkenylene or alkynylene; and n is 0 or 1.

EP 0 533 933 A1

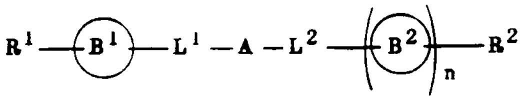

(I)

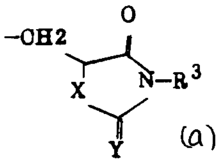

(a)

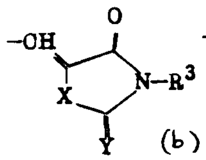

(b)

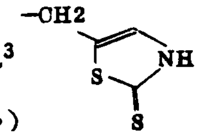

(c)

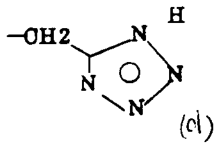

(d)

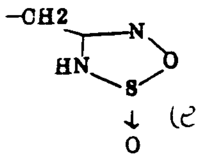

(e)

TECHNICAL FIELD

The present invention relates to novel bishetrocyclic compounds and salts thereof, which are of value as medicines and particularly as antidiabetics.

BACKGROUND ART

The synthetic hypoglycemic agents clinically in use today for the treatment of diabetes are sulfonylureas and biguanides. However, biguanides tend to induce lactic acidosis and are, therefore, restricted in the scope of indication and its use is rather rare today. On the other hand, sulfonylureas are positive in producing antidiabetic effects and have few side effects but as they occasionally induce hypoglycemia, sufficient care must be exercised in their usage.

Much research has been undertaken to develop antidiabetics that may take the place of sulfonylureas but most of development projects were discontinued, with none having been successfully carried into implementation (EP 0 397 453).

Recent years have seen a mounting interest in insulin response potentiators, which enhance the sensitivity of peripheral tissues to insulin and thereby produce hypoglycemic effects, as possible substitutes for the earlier synthetic hypoglycemic agents mentioned above.

However, the insulin response potentiators so far developed are not satisfactory in that they are low in hypoglycemic potency and/or have side effects and, therefore, the development of a drug which would be more potent, with a reduced risk of side effects, has been earnestly in demand.

DISCLOSURE OF THE INVENTION

The inventors of the present invention accordingly created a diversity of compounds and performed an extensive screening. As a result, they found that bisheterocyclic compounds of the following general formula (I), inclusive of salts thereof, produce excellent hypoglycemic effects on account of their insulin response enhancing activity and that the clinical object can be attained by these compounds. The present invention is predicated on the above finding.

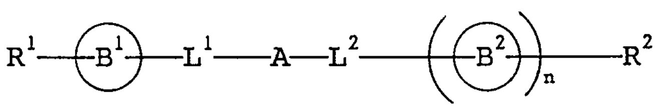 (I)

wherein $R^1$ and $R^2$ may be the same or different and each means a group of the formula

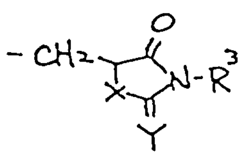 , 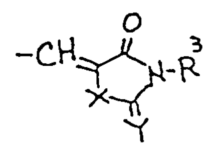 ,

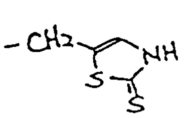 , 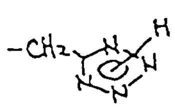 , 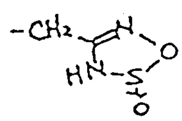

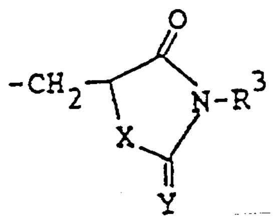 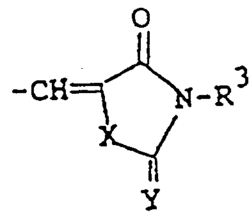 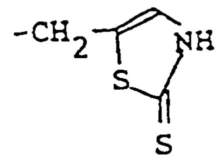

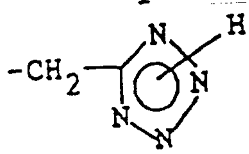 or 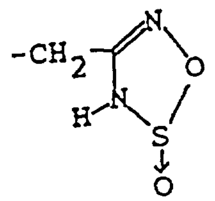

$R^3$ means a hydrogen atom, an acetylamino group, or a lower alkyl group which may be substituted by carboxy or sulfoxy; X and Y are the same or different and each means an oxygen atom, a sulfur atom or a group of the formula NH; $B^1$ and $B^2$ each means a benzene or naphthalene ring which may be substituted by lower alkyl; $L^1$ and $L^2$ are the same or different and each means a single bond, an oxygen atom, a sulfur atom, a sulfonyl group or a sulfinyl group; A means a single bond, a straight-chain or branched alkylene group, a lower $C_2$-$C_6$ alkenylene group or an alkynylene group; n means 0 or 1; provided that where n is equal to 1, $R^1$ and $R^2$ each means

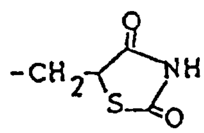 or 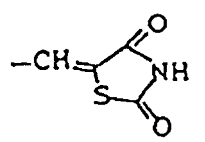 ,

and both of $L^1$ and $L^2$ mean oxygen, a compound wherein A means a straight-chain $C_2$-$C_6$ alkylene group is excluded.

Thus, the present invention is directed to bisheterocyclic compounds of general formula (I), inclusive of salts thereof, and has as its object to provide the same compounds and salts.

The compounds of the invention are described in detail hereinafter.

The "alkylene group" is preferably a straight-chain or branched alkylene group containing 1 to 12 carbon atoms, such as methylene, ethylene, methylmethylene

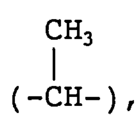

trimethylene, propylene

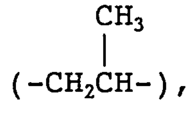

2-propylene

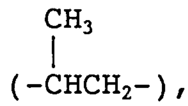

dimethylmethylene

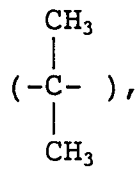

tetramethylene, 1-methyltrimethylene, 2-methyltrimethylene, 3-methyltrimethylene, 1-ethylethylene

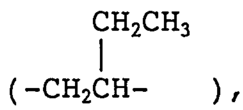

2-ethylethylene

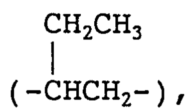

2,2-dimethylethylene

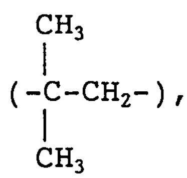

1,1-dimethylethylene

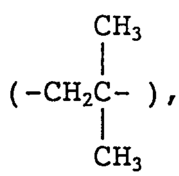

ethylmethylmethylene

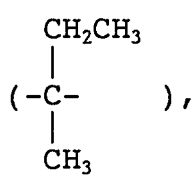

pentamethylene, hexamethylene, heptamethylene, octamethylene, nonamethylene, decamethylene, undecamethylene, dodecamethylene and so on.

The "lower alkylene group" means any of the straight-chain or branched $C_1$-$C_6$ alkylene groups among the above-exemplified species of the "alkylene group".

The lower ($C_2$-$C_6$) alkenylene group means a straight-chain or branched lower alkenylene group containing 2 to 6 carbon atoms.

The alkynylene group is a straight-chain or branched alkynylene group such as -C≡C-,

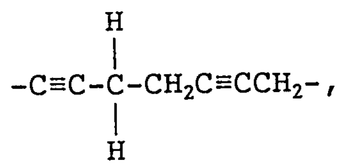

$-CH_2CH_2C{\equiv}CCH_2CH_2-$ and so on.

The compounds (I) of the present invention, inclusive of salts thereof, may have asymmetric carbon atoms and, in such cases, they may occur as isomers. Such optical and other stereoisomers as well as mixtures thereof also fall within the scope of the present invention.

The compound (I) of the present invention has acidic nitrogen in its heterocyclic moieties and may, therefore, form salts with various bases. The present invention covers, within its scope, such salts of compound (I). The salts mentioned just above include salts with various metals such as sodium, potassium, calcium, magnesium, aluminum, etc., salts with organic bases such as methylamine, ethylamine, aminoguanidine, guanidine, 3,5-dimethyl-1-amidinopyrazole, dimethylamine, diethylamine, trimethylamine, triethylamine, monoethanolamine, diethanolamine, triethanolamine, cyclohexylamine, etc., and salts with amino acids such as lysine, ornithine and so on.

The compound (I) of the present invention, inclusive of salts thereof, can be produced by various processes that may be selected according to the structural features of its skeletal moiety and attendant atomic groups. Typical production processes are illustrated below by way of example.

Process 1

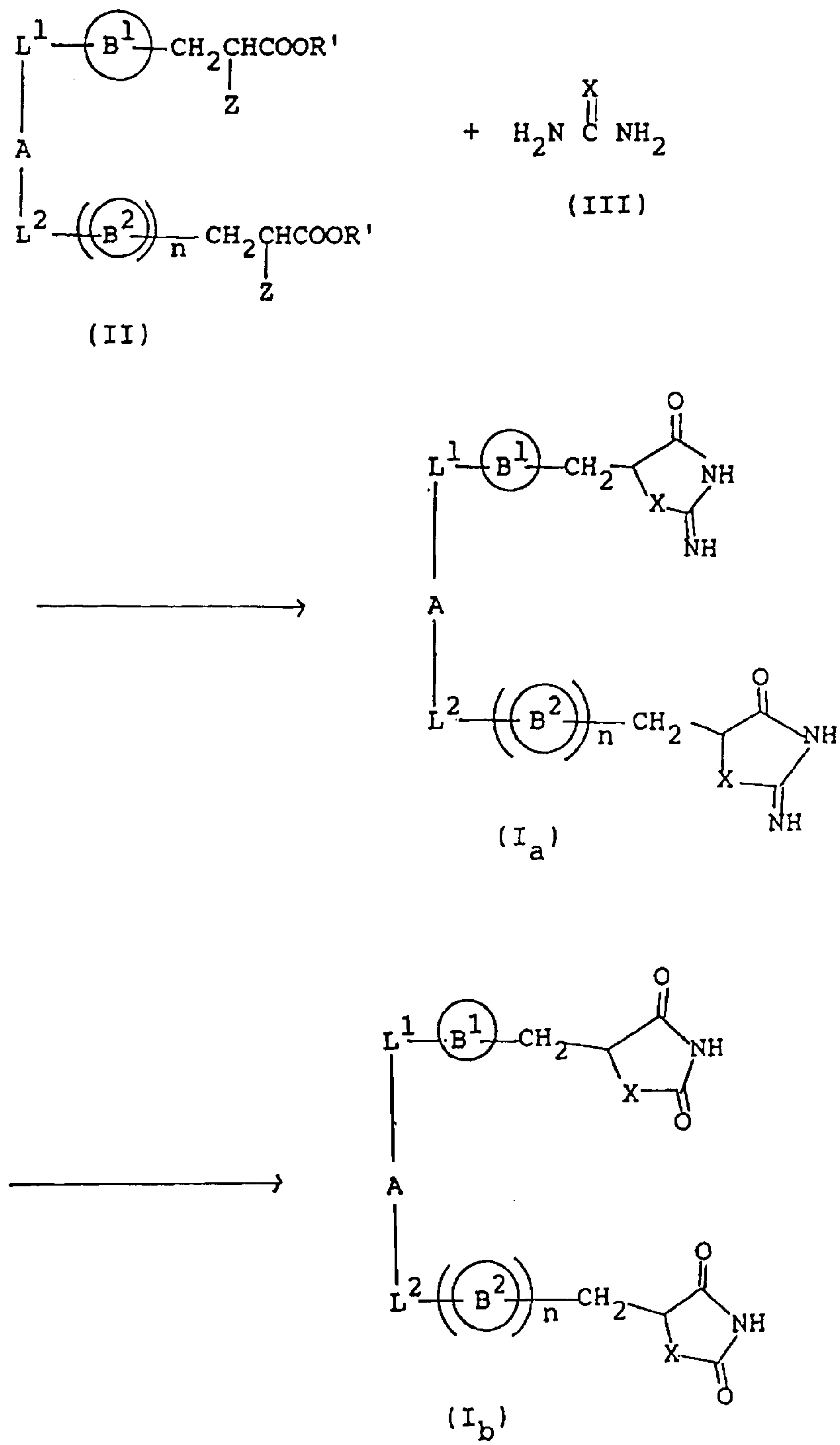

(II) (III) ($I_a$) ($I_b$)

(In the above reaction schema, $B^1$, $B^2$, $L^1$, A, $L^2$ and n are respectively as defined hereinbefore; Z means a halogen atom; R' means a hydrogen atom or an ester residue.)

The bis(iminooxoheterocyclic) derivative of general formula Ia can be produced by reacting a bis-(phenylhalopropionic acid) derivative of general formula II with a compound of the general formula III.

The bis(dioxoheterocyclic) derivative of general formula Ib can be produced by hydrolyzing compound Ia or the reaction mixture containing compound Ia as obtained in the preceding reaction step.

The halogen atom $Z^1$ includes iodine, bromine, chlorine, etc. and the ester residue R' means an ester-forming group, e.g. a lower alkyl group such as methyl, ethyl, propyl, isopropyl, butyl, isobutyl, sec-butyl, tert-butyl, pentyl, isopentyl, neopentyl, sec-pentyl, tert-pentyl, hexyl, isohexyl, etc. or an aralkyl group such as benzyl.

Production of Ia is generally carried out in an inert organic solvent such as an alcohol, e.g. methanol, ethanol, propanol, isopropyl alcohol, methoxyethanol, ethoxyethanol, etc., dimethyl sulfoxide, dimethylformamide or the like. Among them, alcohols are preferred in cases in which the resulting reaction mixture will be directly subjected to the next step for acid hydrolysis.

Since this reaction is a stoichiometric reaction, the starting compounds (II) and (III) can be reacted in equimolar proportions but the compound (III), which is more readily available, may be used in excess.

The reaction is generally conducted with heating and preferably at the reflux temperature of the solvent used.

The reaction is preferably conducted in the presence of the substance which trap hydrogen halide such as sodium acetate or potassium acetate.

The reaction time is decided depending on the particular species of starting compounds and other conditions of reaction.

Production of Ib is carried out in an inert solvent like the one used in the preceding reaction, such as sulfolane, dimethyl sulfoxide, ethoxyethanol, etc., preferably an alcohol, in the presence of excess water and an acid, particularly a strong acid such as hydrochloric acid or hydrobromic acid, generally with heating and preferably under reflux.

The reaction time is decided depending on the particular species of staring compounds and other conditions of reaction.

The starting compound (II) can be obtained, for example as follows.

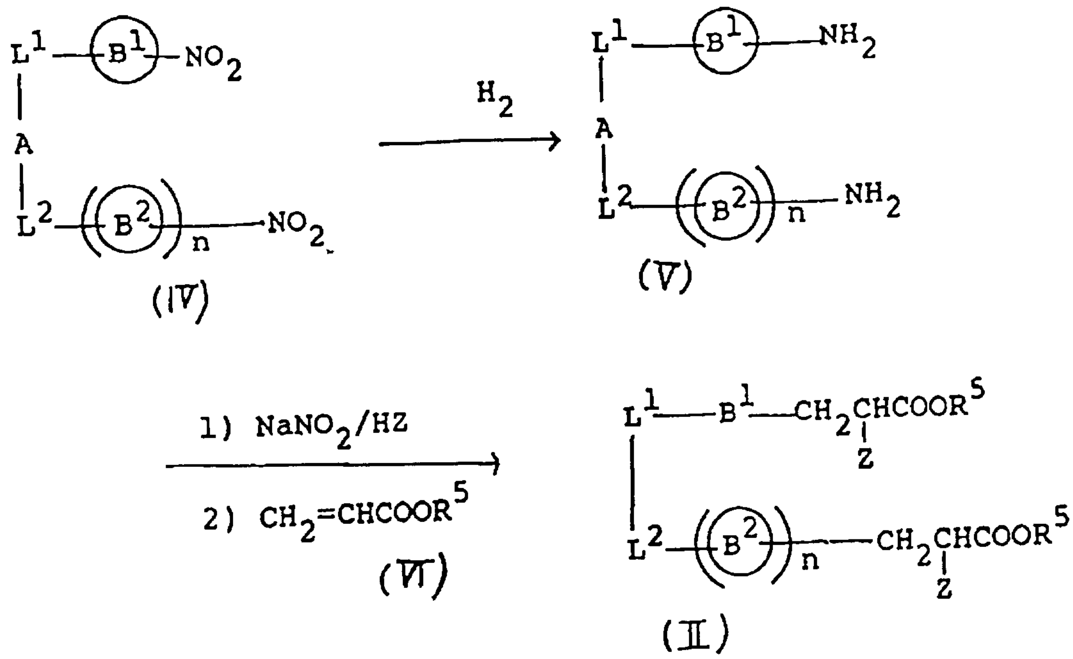


(IV) (V) (VI) (II)

Thus, the starting compound (II) can be prepared by reducing bis(nitrophenyl) compound (IV) to bis-(aminophenyl) compound (V), diazotizing (V) in the presence of a hydrogen halide, and further reacting the diazo compound with acrylic acid or an ester thereof.

The reduction reaction in the first stage is carried out in an inert organic solvent, such as alcohols, acetic acid, etc., by a conventional technique such as catalytic reduction using Raney nickel or palladium-on-carbon catalyst or chemical reaction using a metal, such as zinc or iron, in combination with an acid, such as acetic acid or hydrochloric acid.

The reaction product thus obtained need not be purified but may be directly subjected to the next stage of reaction.

In the next stage, diazotization is carried out using a diazotizing agent, such as sodium nitrite, in an inert organic solvent, e.g. alcohols such as methanol, ethanol, propanol, isopropyl alcohol, methoxyethanol, ethoxyethanol, etc., in the presence of a hydrogen halide, such as hydrochloric acid, hydrobromic acid or hydroiodic acid, at a temperature not exceeding 10 ° C. Then, in the presence of a copper catalyst such as copper(I) oxide, copper(II) oxide, copper chloride, etc., acrylic acid or an ester thereof (VI) is reacted at room temperature. The end point of reaction is confirmed by termination of the evolution of nitrogen gas.

Process 2

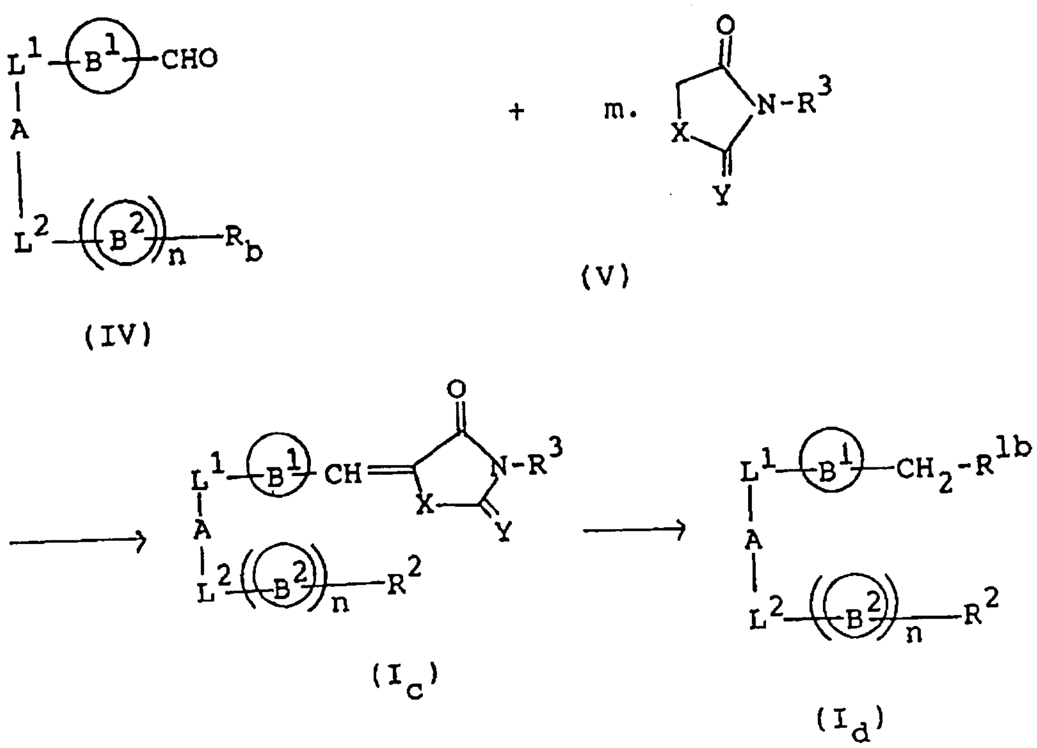

(IV) (V) ($I_c$) ($I_d$)

(In the above reaction schema, $B^1$, $B^2$, $L^1$, A, $L^2$, X, Y, $R^3$ and n are as defined hereinbefore; $R_b$ means a group of the formula -CHO or $R^2$; $R^{1b}$ means a group of the formula

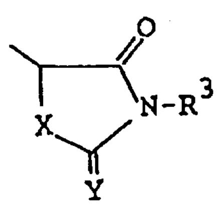 or 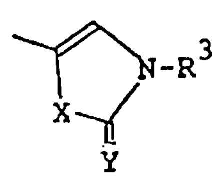 ;

m is equal to 2 when $R^b$ is -CHO, and equal to 1 otherwise.)

The heterocyclic derivative of general formula Ic can be produced by reacting a mono- or bis-aldehyde derivative of general formula IV with a heterocyclic compound of general formula V.

The heterocyclic derivative of general formula Id can be prepared by reducing compound Ic.

The reaction for the production of compound Ic of the present invention is a dehydrative condensation reaction between an aldehyde and an active methylene compound in the presence of a base, which is a well-known reaction called Knoevenagel condensation. While conditions of this reaction are selected according to specific compounds used, the solvent is preferably acetic acid and the particularly preferred

base is ammonium acetate, ammonium chloride or the like. This reaction is conducted generally with heating and preferably under reflux.

The reaction giving rise to compound Id of the present invention resides in reduction of a carbon-carbon double bond, and may for example be hydrogenation with the aid of a catalyst such as palladium-on-carbon or reduction with a hydride such as lithium borohydride, sodium borohydride or the like. The solvent for this reduction reaction may be dimethylimidazolidinone and the preferred reducing agent is sodium borohydride or the like. This reaction is generally conducted under heating.

Reduction of the carbon-carbon double bond with a metal hydride may, depending on reaction conditions, give rise to a group of the formula

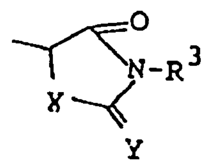

and/or a group of the formula

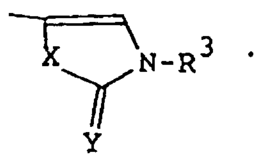

Process 3

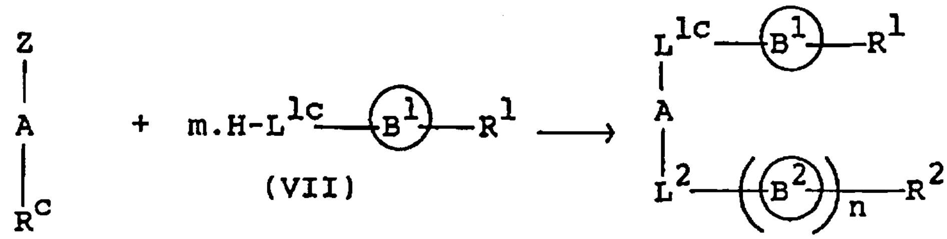

(VII)

(VI)

($I_e$)

(In the above reaction schema, $R^1$, $R^2$, $L^2$, A, $B^1$, $B^2$ and n are as defined hereinbefore; Z means a halogen atom; $R^c$ means a halogen atom or a group of the formula

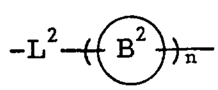

$R^2$; $L^{1c}$ means an oxygen atom or a sulfur atom; m is equal to 2 when $R^c$ is a halogen atom and equal to 1 otherwise (provided, however, that when $R^c$ is a halogen atom, the group

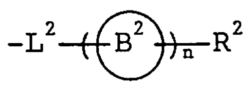

is identical with

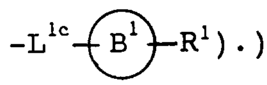

The heterocyclic derivative of general formula Ie can be prepared by reacting an alkyl halide derivative of general formula VI with a phenol or thiophenol compound of general formula VII in the presence of a base. This reaction is a well-known process for synthesis of aromatic ethers or aromatic thioethers. While conditions of this reaction are selected according to specific compounds, the preferred solvent is dimethylformamide and the preferred base is potassium carbonate. Depending on the type of base, the reaction is conducted under cooling. Generally, however, this reaction is carried out at room temperature or under heating.

Process 4

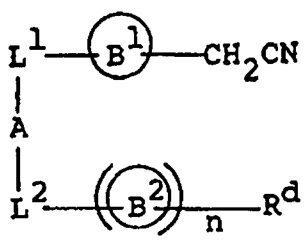

(VIII)

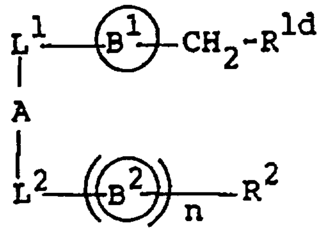

($I_f$)

($R^2$, $L^1$, $L^2$, A, $B^1$, $B^2$ and n are as defined hereinbefore; $R^d$ means a cyanomethyl group or a group represented by $R^2$; $R^{1d}$ means

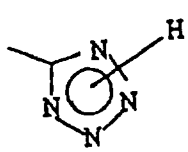

or

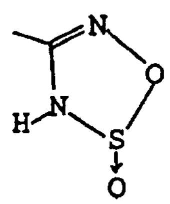

;

provided, however, that when $R^d$ is a cyanomethyl group; $R^2$ is identical with $-CH_2-R^{1d}$ mentioned hereinbefore.)

The heterocyclic derivative of general formula If can be produced from a mono- or bisnitrile derivative of general formula VIII by a well-known reaction for converting a cyano group to a heterocyclic group.

The reaction for conversion of a cyano group to a group of the formula

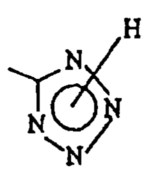

is conducted, for example by heating a mixture of nitrile derivative VIII, sodium azide and ammonium chloride using dimethylformamide as the solvent. The reaction for conversion of a cyano group to a group of the formula

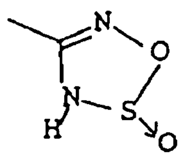

can be conducted, for example by heating a mixture of nitrile derivative VIII, sodium methylate and hydroxylamine hydrochloride using methanol as the solvent to convert the cyano group to

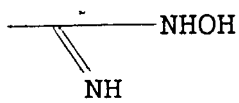

and, then, reacting the conversion product with pyridine and thionyl chloride in the solvent dichloromethane.

Process 5

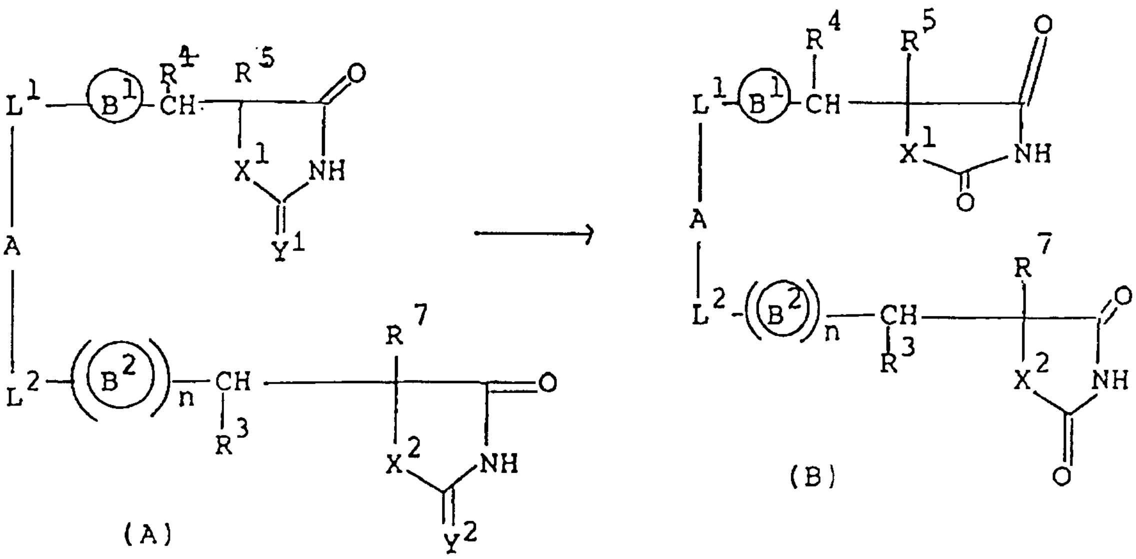

(In the above reaction schema, $B^1$, $B^2$, $L^1$, $L^2$, A and n are as defined hereinbefore; $R^4$, $R^5$, $R^6$ and $R^7$ each means a hydrogen atom or an alkyl group or $R^4$, $R^5$ and/or $R^6$, $R^7$ jointly mean a single bond; $X^1$ and $X^2$ are the same or different and each means an oxygen atom, a sulfur atom or NH; $Y^1$ and $Y^2$ are the same or different and each means an oxygen atom or a sulfur atom but both of $Y^1$ and $Y^2$ cannot be oxygen atoms.)

The bisheterocyclic derivative of general formula (B) can be produced by treating a bisheterocyclic derivative of general formula (A) with bromine, a peroxide or the like.

This reaction is a well-known process for the conversion of a thiocarbonyl group to a carbonyl group. While conditions of the reaction are selected depending on specific compounds, the solvent may for example be acetic acid, methoxyethanol, ethoxyethanol, dimethylformamide or N,N'-dimethylimidazolidinone, although bromine may be used in excess so that it may double as the reactant and the solvent.

Depending on particular species of compounds used, the reaction proceeds under heating. Generally, however, this reaction is carried out under cooling or at room temperature.

Process 6

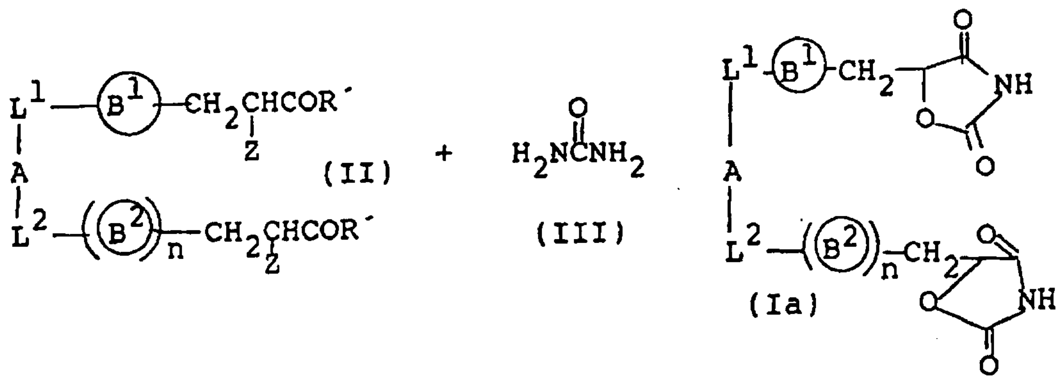

(II) (III) (Ia)

(In the reaction schema, $B^1$, $B^2$, $L^1$, A, $L^2$ and n are as defined hereinbefore; Z means a hydroxy group, $R^1$ means an amide residue or an ester residue.)

The bis(dioxooxazaline) derivative of general formula (Ia) can be produced by reacting a bis-(phenylhydroxypropionic acid) derivative of general formula (II) with urea or a dialkyl carbonate in the presence of a base such as sodium methoxide, potassium butoxide or the like.

Other processes

Some of the compounds of the present invention may be subjected to further reactions to give other object compounds. These reactions can be carried out by the following or other well-known processes.

a) The sulfide compound in which at least one of $L^1$ and $L^2$ is a sulfur atom can be oxidized by a well-known oxidation method to the sulfinyl compound in which said sulfur atom exists in the form of

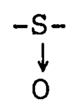

and further to the sulfone compound in which said sulfur atom exists in the form of $-SO_2-$.

b) Where at least one of $R^1$ and $R^2$ is a group of the formula

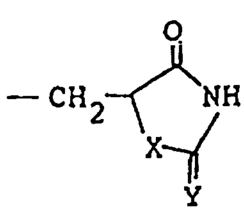

or

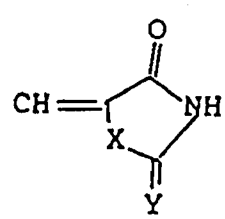

,

the well-known N-alkylation reaction can be conducted to obtain the object compound having a group of the formula

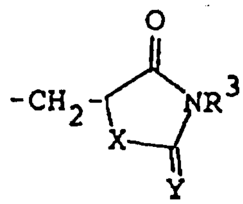

or

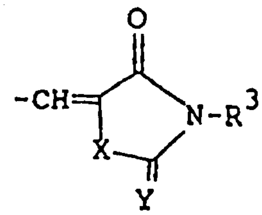

The compound (I) of the invention, produced as above, can be isolated and purified in the free form or in the form of a salt.

The isolation and purification of the compound can be performed by the well-known chemical procedures, such as extraction, crystallization, recrystallization and various column chromatographies, particularly silica gel column chromatography.

(Effect of the Invention)

The compound (I) and salts thereof of the present invention have exceedingly high hypoglycemic potencies due to their action to potentiate the sensitivity to insulin and are low in toxicity. Therefore, they are of use as prophylactic and therapeutic agents for diabetes, particularly non-insulin-dependent diabetes mellitus (type II), and various diabetic complications, as well as for combination therapy with insulin.

The hypoglycemic potency of the compounds of the invention, which is derived from their action to enhance the sensitivity to insulin was confirmed by the following test procedure.

Hypoglycemic activity

Male KK mice, 4-5 weeks old, were obtained from Japan Clea. The animals were individually put on a high caloric diet (CMF, Oriental Yeast) and the individuals weighing about 40 g were selectively used.

For determination of blood glucose, 10 μl of blood was withdrawn from the caudal vein, deproteinized with 100 μl of 0.33 N perchloric acid and centrifuged. The glucose in the supernatant was assayed by the glucose oxidase method. Groups of 6 animals showing 200 mg/dℓ or more of blood glucose were submitted to the experiment.

The test compound was suspended in 0.5% methylcellulose and administered daily per os for 4 consecutive days. Before administration and on day 5, the blood was withdrawn from the caudal vein of each animal and the blood glucose was determined by the above method.

Hypoglycemic potency was expressed in terms of percent decrease from the glucose level prior to administration of the test compound and the results were statistically evaluated at the significance level of $p = 0.05$.

* = $p<0.05$
** = $p<0.01$
*** = $p<0.001$

As a result, a significant hypoglycemic effect was attained within the dose range of 10 to 300 mg/kg.

The inhibition rates at the dosage level of 100 mg are shown in the following table.

| Compound | % Inhibition |
|---|---|
| Compound of Example 14 | 37% |
| Compound of Example 15 | 47% |
| Compound of Example 16 | 51% |

The compounds of the present invention are low in toxicity and when the compound of Example 15, for instance, was orally administered to Fischer rats (2 animals per sex) in doses of 1,000 mg/kg for 3 consecutive days, no abnormality was observed.

A pharmaceutical composition containing one or more species of the compound of the general formula (I) or salts thereof as the active ingredient can be manufactured using the carrier, excipient and/or other additives which are commonly used in pharmaceutical production.

The carrier and excipient mentioned above include solid or liquid non-toxic substances for pharmaceutical use, such as lactose, magnesium stearate, starch, talc, gelatin, agar, pectin, gum arabic, olive oil,

sesame oil, cacao butter, ethylene glycol and other substances which are in common usage.

Like the conventional synthetic hypoglycemic drugs such as sulfonylurea preparations, the pharmaceutical composition of the invention can advantageously provided in oral dosage forms such as the tablet, capsule, powder, fine granule, granule, pill, etc. for avoiding inconveniences such as those experienced with insulin injections. Of course, the composition may be any other type of preparation for non-oral administration such as injections, suppositories, transdermal therapeutic systems (TTS) (inclusive of those for buccal application), transnasal therapeutic systems and so on.

The clinical dosage of the compound of the invention depends on the patient's condition, body weight, age and sex, among other factors. In the case of oral administration, the daily dosage for an adult patient may range from 10 to 2,000 mg, which is to be administered in a single dose or in two or several divided doses.

(Examples)

The following examples are further illustrative of the present invention.

Some of the starting compounds used in the practice of the invention are novel compounds. Therefore, processes for production of such compounds are described below in Reference Examples.

When a process for producing any starting material and a process for producing a compound of the invention from that starting material were carried out in a continuous sequence, the former process is described in the relevant Example.

Reference Example 1

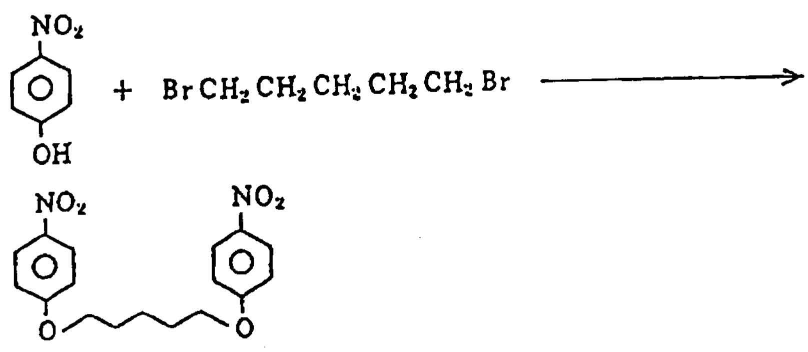

A mixture of 500 ml of dimethylformamide, 46 g (0.2 mol) of 1,5-dibromopentane, 55.6 g (0.4 mol) of 4-nitrophenol and 60 g of $K_2CO_3$ was stirred at 60-70 ° C for 8 hours. After completion of the reaction, 300 ml of water was added and the resulting crystals were collected by filtration and washed with methanol to give 70 g of 1,5-bis(p-nitrophenoxy)pentane.

Example 1

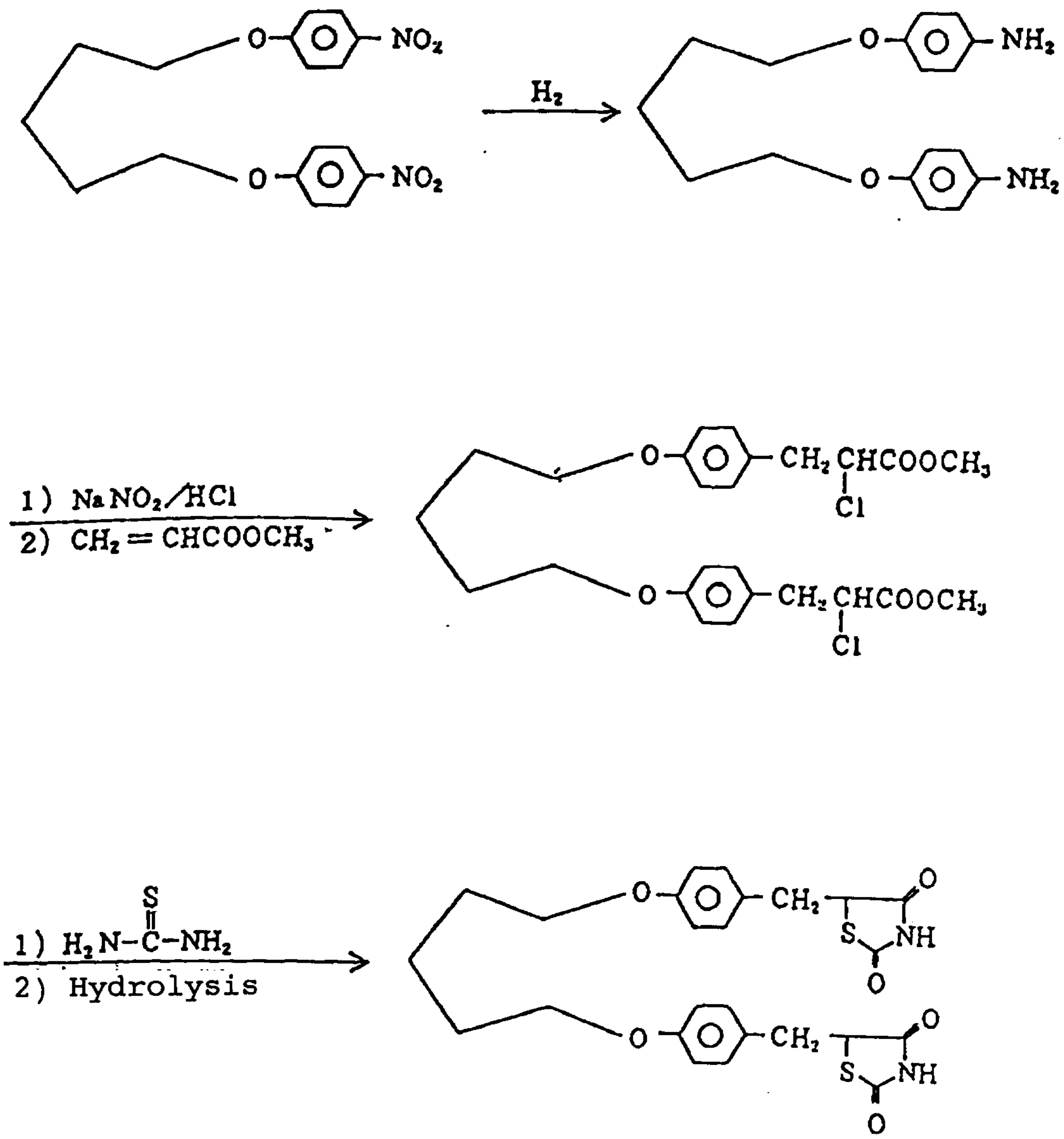


In 1 ℓ of acetic acid, 34.6 g (0.1 mol) of 1,5-bis(p-nitrophenoxy)pentane was subjected to hydrogenation in the presence of 10 ml of Raney nickel catalyst and the acetic acid was then distilled off. The residue was dissolved in 2 ℓ of methanol. To this solution was added 200 ml of concentrated hydrochloric acid and the mixture was cooled to 0 °C. While this mixture was stirred, a saturated aqueous solution of 13.8 g (0.2 mol) of sodium nitrite was added dropwise at a temperature not exceeding 10 °C. After completion of the reaction, 300 g of methyl acrylate was added and 10 g of copper(I) oxide was added gradually. The mixture was allowed to stand at room temperature overnight. After the evolution of nitrogen gas had terminated, the reaction mixture was concentrated to dryness under reduced pressure. The residue was dissolved in water (1ℓ)-ether (2ℓ) and the ether layer was separated and dried over anhydrous magnesium sulfate. The ether was then distilled off and the residue was dissolved in 200 ml of ethanol. To the solution were added 15.2 g (0.2 mol) of thiourea and 16.5 g (0.2 mol) of sodium acetate and the mixture was stirred on an oil bath at 140 °C for 15 hours. Thereafter, 100 ml of 4N-hydrochloric acid was added and the mixture was further stirred on an oil bath at 140 °C for 15 hours. The ethanol was then distilled off under reduced pressure and 500 ml of water and 500 ml of ethyl acetate were added. The ethyl acetate layer was separated and dried over anhydrous magnesium sulfate and the ethyl acetate was distilled off. The residue was subjected to silica gel column chromatography (eluent: chloroform containing 2% of methanol) and the eluate corresponding to Rf = 0.11 was pooled to give 8 g of 1,5-bis[p-[(2,4-dioxo-5-thiazolidinyl)methyl]phenoxy]-

pentane.

Byproducts were detected at Rf = 0.56 and Rf = 0.43.

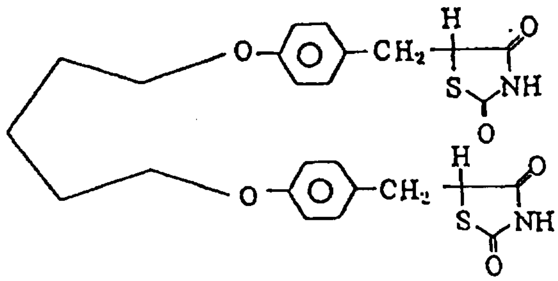

1,5-Bis[p-[(2,4-dioxo-5-thiazolidinyl)methyl]phenoxy]pentane

Starting compound: 1,5-Bis(p-nitrophenoxy)pentane

Physicochemical properties

Rf = 0.11

Melting point 143-145 ° C, isopropyl alcohol

| Elemental analysis (for $C_{25}H_{26}N_2O_6S_2$) | | | | |
|---|---|---|---|---|
| | C (%) | H (%) | N (%) | S (%) |
| Calcd. | 58.35 | 5.09 | 5.44 | 12.46 |
| Found | 58.31 | 5.10 | 5.33 | 12.46 |

Mass spectrum (m/z): 513 $(M-1)^-$ Fab (Neg.)

Nuclear magnetic resonance spectrum ($d_6$-DMSO, TMS internal standard)

δ: 1.4-2.0 (6H, m, $-CH_2-\underline{(CH_2)_3}-CH_2-$),

3.02 (2H, dd, 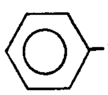-C<u>H</u>H-), 3.30 (2H, dd, 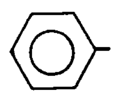-CH<u>H</u>-),

3.93 (4H, brt, $-O-CH_2-$),

4.83 (2H, dd, -CH-),

6.75-8.2 (8H, m, phenyl), 11.98 (2H, br, NH)

Examples 2-5

The following compounds were obtained in substantially the same manner as described in Example 1.

Example 2

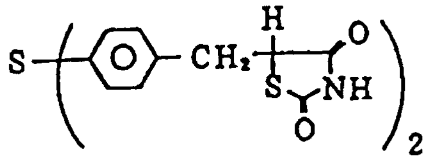

Bis[p-[(2,4-dioxo-5-thiazolidinyl)methyl]phenyl] sulfide

Physicochemical properties

Rf = 0.09

Melting point 188-189 ° C, ether

| Elemental analysis (for $C_{20}H_{16}N_2O_4S_3$) | | | | |
|---|---|---|---|---|
| | C (%) | H (%) | N (%) | S (%) |
| Calcd. | 54.04 | 3.63 | 6.30 | 21.64 |
| Found | 54.98 | 3.84 | 5.79 | 19.79 |

Mass spectrum (m/z): 444 ($M^+$)

Nuclear magnetic resonance spectrum ($d_6$-DMSO, TMS internal standard)

δ: 3.10 (2H, dd, -CHH-),

4.89 (2H, dd, -CH-),

7.2-7.3 (8H, s, phenyl)

Example 3

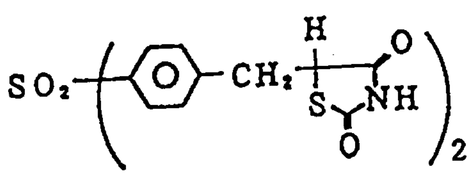

Bis[p-[(2,4-dioxo-5-thiazolidinyl)methyl]phenyl]sulfone

Physicochemical properties

Rf = 0.03

Melting point 224-231 ° C, isopropyl alcohol

| Elemental analysis (for $C_{20}H_{16}N_2O_6S_3$) | | | | |
|---|---|---|---|---|
| | C (%) | H (%) | N (%) | S (%) |
| Calcd. | 50.41 | 3.38 | 5.88 | 20.19 |
| Found | 50.29 | 3.37 | 5.79 | 19.86 |

Mass spectrum (m/z): 476 ($M^+$), 360, 254

Nuclear magnetic resonance spectrum ($d_6$-DMSO, TMS internal standard)

δ: 3.1-3.7 (4H, m, $-CH_2-$),

4.8-5.1 (2H, m, -CH-),

7.4-8.1 (8H, m, phenyl), 12.1 (2H, br, NH)

Example 4

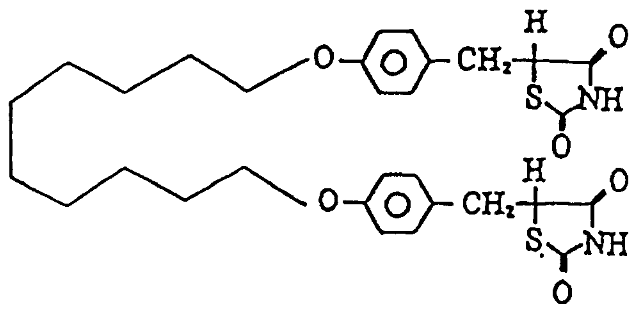

1,10-Bis[p-[(2,4-dioxo-5-thiazolidinyl)methyl]phenoxy]decane

Starting compound: 1,10-Bis(p-nitrophenoxy)decane

Physicochemical properties

Rf = 0.18

Melting point 139-142 °C, isopropyl alcohol

| Elemental analysis (for $C_{30}H_{36}N_2O_6S_2$) | | | | |
|---|---|---|---|---|
| | C (%) | H (%) | N (%) | S (%) |
| Calcd. | 61.61 | 6.21 | 4.79 | 10.97 |
| Found | 61.65 | 6.28 | 4.63 | 10.72 |

Mass spectrum (m/z): 583 $(M-1)^-$ Fab (Neg.)

Nuclear magnetic resonance spectrum ($d_6$-DMSO, TMS internal standard)

δ: 1.0-1.9 (16H, m, O-$CH_2$-($\underline{CH_2}$)$_8$-$CH_2$-O),

3.02 (1H, dd, 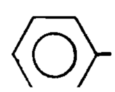-C$\underline{H}$H-),

3.92 (4H, t, -O-$CH_2$-),

4.86 (2H, dd, -CH-),

6.8-7.3 (8H, m, phenyl), 11.84 (2H, br, NH)

Example 5

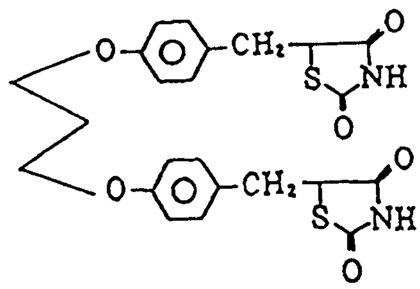

1,3-Bis[p-[(2,4-dioxo-5-thiazolidinyl)methyl]phenoxy]propane
Starting compound: 1,3-Bis(p-nitrophenoxy)propane
Physicochemical properties
Rf = 0.13
Melting point 147-148 ° C, methanol

| Elemental analysis (for $C_{23}H_{22}N_2O_6S_2$) | | | | |
|---|---|---|---|---|
| | C (%) | H (%) | N (%) | S (%) |
| Calcd. | 56.78 | 4.56 | 5.76 | 13.18 |
| Found | 56.81 | 4.68 | 5.55 | 12.91 |

Mass spectrum (m/z): 485 $(M-1)^-$ Fab (Neg.)
Nuclear magnetic resonance spectrum ($d_6$-DMSO, TMS internal standard)
δ: 1.96-2.30 (2H, m, $-CH_2-(CH_2)_3-CH_2-$),

3.02 (2H, dd, 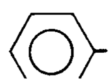-CHH-),

4.07 (4H, brt, $-OCH_2-$),

4.83 (2H, dd, -CH-),

6.7-8.2 (8H, m, phenyl), 12.0 (1H x 2, br, NH)

Example 6

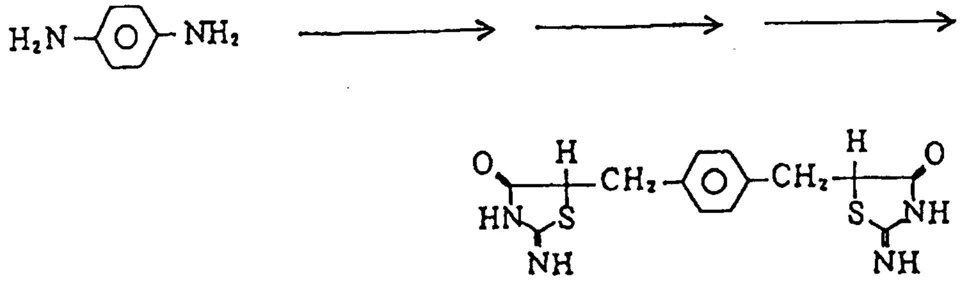

In 200 ml of methanol was dissolved 21.6 g (0.2 mol) of p-phenylenediamine followed by addition of 100 ml of concentrated hydrochloric acid, and the mixture was cooled to 0 ° C. While this mixture was stirred, a saturated aqueous solution of 27.6 g (0.04 mol) of sodium nitrite was added dropwise at a temperature not exceeding 10 ° C. After completion of the reaction, 103 g of methyl acrylate was added and 10 g of copper(I) oxide was added gradually. The mixture was allowed to stand at room temperature overnight. After the evolution of nitrogen gas had terminated, the reaction mixture was concentrated to dryness under reduced pressure and the residue was diluted with 500 ml of water and ether. The ether layer was separated and dried over anhydrous magnesium sulfate. The ether was then distilled off and the residue was dissolved in 100 ml of ethanol. Then, 30.4 g (0.4 mole) of thiourea and 33 g (0.4 mol) of sodium acetate were added and the mixture was stirred on an oil bath at 140 ° C for 15 hours. The resulting precipitate was recovered by filtration, washed thoroughly with methanol and water and dried to give 40 g of crude 1,4-bis[(2-imino-4-oxo-5-thiazolidinyl)methyl]benzene.

Physicochemical properties

Nuclear magnetic resonance spectrum ($d_6$-DMSO, TMS internal standard)

δ: 2.85 (2H, dd, -CHH-),

4.55 (2H, dd, -CH-),

7.16 (4H, s, phenyl), 9.01 (4H, br, NH)

Example 7

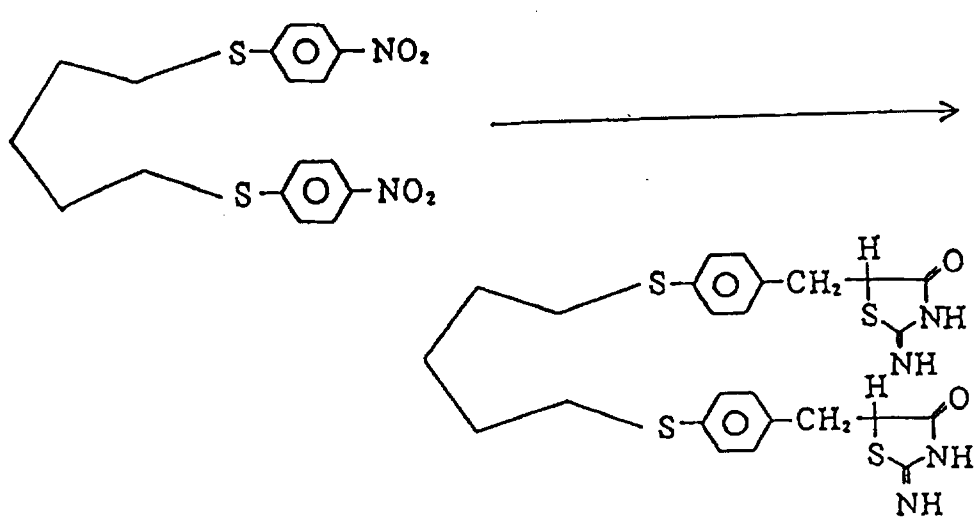

In 100 ml of acetic acid, 7.56 g (0.02 mole) of 1,5-bis[(p-nitrophenyl)thio]pentane was subjected to hydrogenation in the presence of 2 ml of Raney nickel catalyst and the acetic acid was then distilled off. The residue was dissolved in 50 ml of methanol and after addition of 10 ml of concentrated hydrochloric acid, the solution was cooled to 0 ° C. While this solution was stirred, a saturated aqueous solution of 2.76 g (0.04 mol) of sodium nitrite was added dropwise at a temperature not exceeding 10 ° C. After completion of the reaction, 10.3 g of methyl acrylate was added and, then, 1 g of copper(I) oxide was added gradually. The mixture was allowed to stand at room temperature overnight. After the evolution of nitrogen gas had terminated, the reaction mixture was concentrated to dryness under reduced pressure. The residue was dissolved in water (100 ml)-ether (100 ml) and the ether layer was separated and dried over anhydrous magnesium sulfate. The ether was then distilled off and the residue was dissolved in 50 ml of ethanol, followed by addition of 3.04 g (0.04 mol) of thiourea and 3.3 g (0.04 mol) of sodium acetate. The mixture was stirred on an oil bath at 140 ° C for 15 hours. The resulting precipitate was recovered by filtration, washed thoroughly with methanol and water and dried to give 4.2 g of crude 1,5-bis[[p-[(2-imino-4-oxo-5-thiazolidinyl)methyl]phenyl]thio]pentane.

Physicochemical properties

Nuclear magnetic resonance spectrum ($d_6$-DMSO, TMS internal standard)

δ: 1.4-1.7 (6H,m,$-CH_2-(CH_2)_3-CH_2-$), 2.6-3.1 (6H, m, -CHH-, $-S-CH_2-$), 3.34 (2H, dd, -CHH-),

4.55 (2H, dd, -CH-),

7.2 (8H, s, phenyl), 9.0 (2H, br, NH)

Example 8

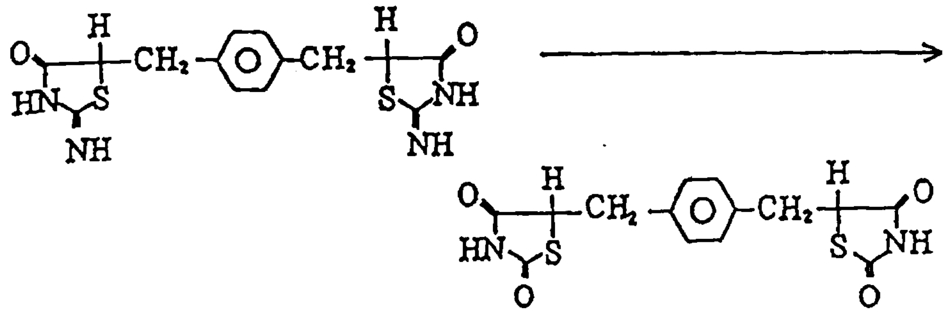

In 50 ml of ethanol was dissolved 40 g of the crude 1,4-bis[(2-imino-4-oxo-5-thiazolidinyl)methyl]-benzene obtained in Example 6, followed by addition of 100 ml of 4N-hydrochloric acid, and the mixture was stirred on an oil bath at 140°C for 15 hours. After cooling to room temperature, the resulting crystals were collected by filtration and washed with methanol to give 25 g of 1,4-bis[(2,4-dioxo-5-thiazolidinyl)-methyl]benzene.

Physicochemical properties

Melting point 257-259°C, ethanol-water

| Elemental analysis (for $C_{14}H_{12}N_2O_4S_2$) | | | | |
|---|---|---|---|---|
| | C (%) | H (%) | N (%) | S (%) |
| Calcd.<br>Found | 49.99<br>50.04 | 3.60<br>3.58 | 8.33<br>8.22 | 19.06<br>19.02 |

Mass spectrum (m/z): 336 ($M^+$), 275, 220

Nuclear magnetic resonance spectrum ($d_6$-DMSO, TMS internal standard)

δ: 3.12 (2H, dd, -CHH-), 3.42 (2H, dd, -CHH-),

4.94 (2H, dd, -CH-),

7.28 (4H, s, phenyl), 12.0 (2H, br, NH)

Example 9

The following compound was obtained in substantially the same manner as in Example 8.

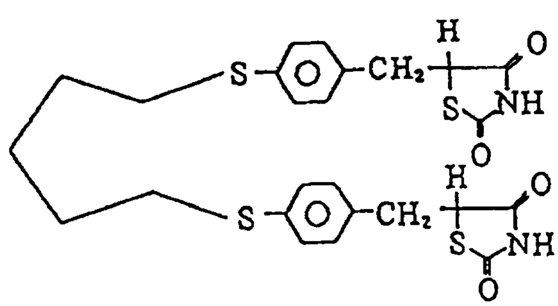

1,5-Bis[[p-[(2,4-dioxo-5-thiazolidinyl)methyl]phenyl]thio]pentane
Starting compound: The compound of Example 7
Physicochemical properties
Melting point 120-123 ° C, methanol

| Elemental analysis (for $C_{25}H_{26}N_2O_4S_4$) | | | | |
|---|---|---|---|---|
| | C (%) | H (%) | N (%) | S (%) |
| Calcd. | 54.92 | 4.79 | 5.12 | 23.46 |
| Found | 54.82 | 4.81 | 5.02 | 23.23 |

Mass spectrum (m/z): 545 $(M-1)^-$ Fab (Neg.)
Nuclear magnetic resonance spectrum ($d_6$-DMSO, TMS internal standard)
δ: 1.5-1.8 (6H, m, $SCH_2$-$\underline{(CH_2)_3}$-$CH_2$-S-), 2.8-3.2 (4H, m, -S-$CH_2$-),

3-3.6 (4H, m, 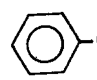$CH_2$-),

4.89 (2H, dd, -CH-),

7.1-7.4 (8H, brs, phenyl), 12.05 (2H, br, NH)

Example 10

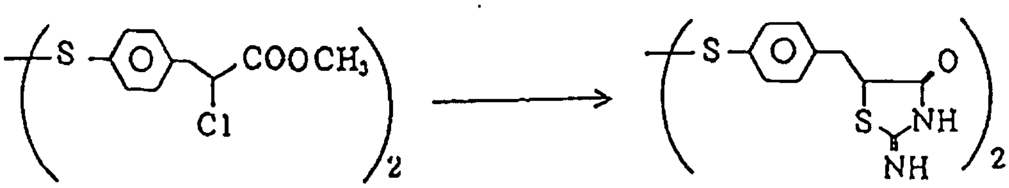

In ethanol (200 ml) were dissolved 8.7 g (0.019 mol) of bis[4-(2-chloro-2-methoxycarbonylethyl)phenyl] disulfide, 3.46 g (0.046 mol) of thiourea and 3.73 g (0.046 mol) of sodium acetate and the solution was refluxed for 63 hours. After completion of the reaction, the precipitate was recovered by filtration, washed successively with ethanol, water, ethanol and ethyl acetate, and dried under reduced pressure. The procedure gave 8.75 g (97%) of bis[4-[(2-imino-4-oxo-5-thiazolidinyl)methyl]phenyl] disulfide.
Physicochemical properties
Melting point 235-237 ° C
Mass spectrum (m/z): 475 $(M+1)^+$ Fab (pos.)
Nuclear magnetic resonance spectrum ($d_6$-DMSO, TMS internal standard)
δ: 2.89 (1H, dd, -C$\underline{H}$H-), 3.34 (1H, dd, -CH$\underline{H}$-), 4.55 (1H, dd, -CH-), 7.20 (2H, d, phenyl), 7.40 (2H, d, phenyl), 8.66 (1H, br, NH), 8.88 (1H, br, NH)

Example 11

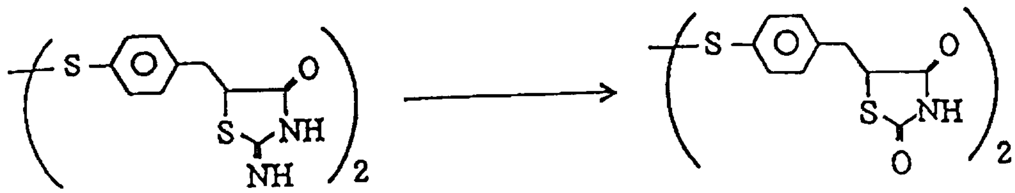

To 8.65 g (0.018 mol) of bis[4-[(2-imino-4-oxo-5-thiazolidinyl)methyl]phenyl] disulfide were added 100 ml of ethanol, 100 ml of pure water and 100 ml of concentrated hydrochloric acid and the mixture was refluxed for 16 hours. The reaction mixture was then cooled, neutralized with sodium hydrogen carbonate and extracted with ethyl acetate. The extract was washed with a saturated aqueous solution of sodium chloride and dried over anhydrous magnesium sulfate. The solvent was then distilled off and the residue was purified by column chromatography (chloroform-methanol = 100:1) to give 5.96 g (69%) of bis[4-[(2,4-dioxo-5-thiazolidinyl)methyl]phenyl] disulfide.

Physicochemical properties

Melting point 73-74 ° C

| Elemental analysis (for $C_{20}H_{16}N_2O_4S_4$) | | | | |
|---|---|---|---|---|
| | C (%) | H (%) | N (%) | S (%) |
| Calcd. | 50.40 | 3.38 | 5.88 | 26.91 |
| Found | 50.49 | 3.48 | 5.65 | 26.80 |

Mass spectrum (m/z): 475 $(M-1)^-$ Fab (Neg.)

Nuclear magnetic resonance spectrum ($d_6$-DMSO, TMS internal standard)

δ: 3.03 (2H, dd, CHH), 3.52 (2H, dd, CHH),

4.84 (2H, dd, -CH-),

7.18 (4H, d, phenyl), 7.40 (4H, d, phenyl), 12.0 (2H, brs, NH),

Example 12

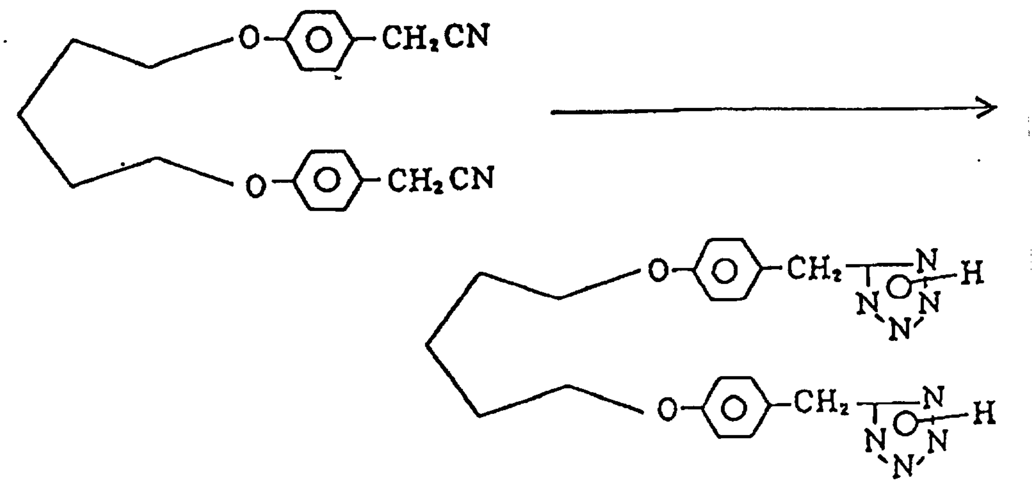

In 50 ml of dimethylformamide were dissolved 3.34 g (0.01 mol) of 1,5-bis(4-cyanomethylphenoxy)-pentane, 1.30 g (0.02 mol) of sodium azide and 1.07 g (0.02 mol) of ammonium chloride and the solution was stirred on an oil bath at 130°C overnight. After completion of the reaction, the solvent was distilled off under reduced pressure and the residue was diluted with water. The resulting crystals were collected by filtration and recrystallized from ethanol to give 1.85 g of 1,5-bis[[4-(5-tetrazolyl)methyl]phenoxy]pentane.

Physicochemical properties

Melting point 206-207°C (dec.) ethanol

| Elemental analysis (for $C_{21}H_{24}N_8O_2 \cdot 0.8H_2O$) | | | |
|---|---|---|---|
| | C (%) | H (%) | N (%) |
| Calcd. | 58.00 | 5.93 | 25.77 |
| Found | 57.83 | 5.65 | 25.55 |

Mass spectrum (m/z): 419 $(M-1)^-$ Fab (Neg.)

Nuclear magnetic resonance spectrum ($d_6$-DMSO, TMS internal standard)

δ: 1.30-1.90 (6H, m, $-CH_2-(\underline{CH_2})_3-CH_2$), 3.80-4.10 (4H, m, $-O\underline{CH_2}-$), 4.16 (4H, s, $-CH_2-Ph$), 3.70-7.30 (8H, m, phenyl)

Example 13

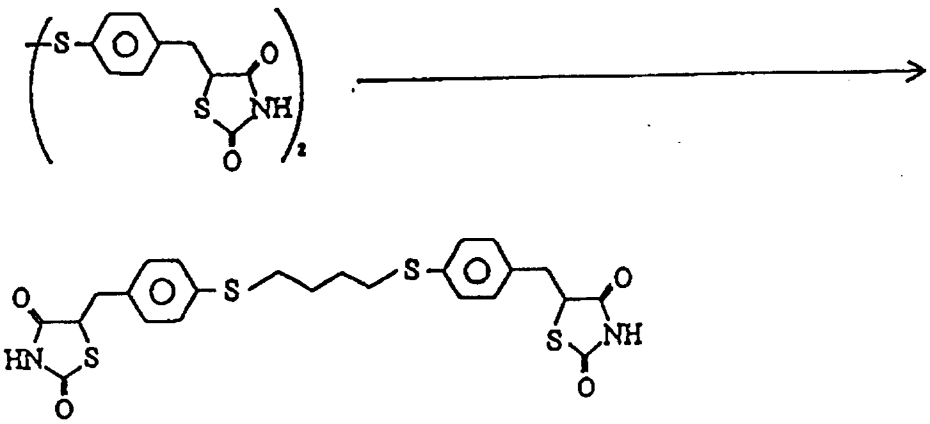

To 2.5 g of bis[4-[(2,4-dioxo-5-thiazolidinyl)methyl]phenyl] disulfide were added 21 ml of 1,4-dioxane, 5.2 ml of pure water, 1.3 g (0.005 mol) of triphenylphosphine and one drop of concentrated hydrochloric acid and the mixture was stirred in an argon atmosphere at 40 ° C for 20 minutes. After completion of the reaction, the solvent was distilled off under reduced pressure and the residue was diluted with benzene and dried over anhydrous magnesium sulfate. The solvent was then distilled off and the residue was dissolved in 40 ml of dimethylformamide. To this solution were added 0.91 g (0.004 mol) of 1,4-dibromobutane and 0.73 g (0.005 mol) of potassium carbonate and the mixture was stirred at room temperature for 1 hour. The insoluble matter was filtered off and the solvent was distilled off under reduced pressure. The residue was dissolved in ethyl acetate, rinsed with water and dried over anhydrous magnesium sulfate. The solvent was then distilled off and the residue was purified by column chromatography (chloroform-methanol = 50:1). The resulting yellow oil was crystallized from chloroform and the crystals thus obtained were washed with hot methanol and chloroform to give 0.51 g (22.7%) of 1,4-bis[[4-[(2,4-dioxo-5-thiazolidinyl)methyl]phenyl]thio]-butane.

Physicochemical properties

Melting point 174-176 ° C

| Elemental analysis (for $C_{24}H_{24}N_2O_4S_4$) | | | | |
|---|---|---|---|---|
| | C (%) | H (%) | N (%) | S(%) |
| Calcd. | 54.11 | 4.54 | 5.26 | 24.08 |
| Found | 54.25 | 4.54 | 5.17 | 24.13 |

Mass spectrum FAB (Pos.)(m/z): 533 $(M+1)^+$

Nuclear magnetic resonance spectrum ($d_6$-DMSO, TMS internal standard)

δ:

1.66 (4H, brs, $-CH_2-(CH_2)_2-CH_2-$),

2.96 (4H, brs, -S-C<u>H</u>$_2$), 3.09 (2H, dd, -C<u>H</u>H-CH< ), 4.91 (2H, dd, -CH< ), 7.20 (4H, d, phenyl), 7.26 (4H, d, phenyl)

Example 14

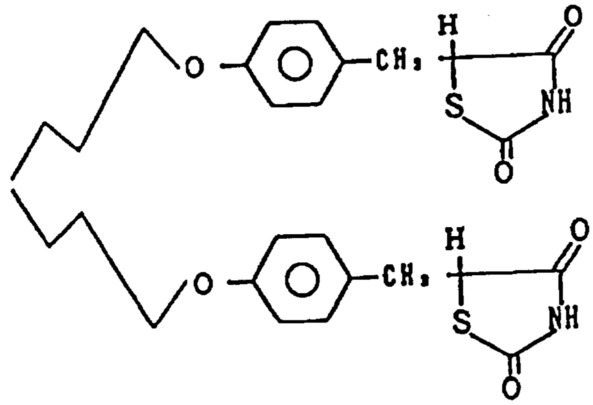

In 1ℓ of acetic acid, 37.4 g (0.1 mol) of 1,7-bis(p-nitrophenoxy)heptane was subjected to hydrogenation in the presence of 10 ml of Raney nickel catalyst and the acetic acid was then distilled off. The residue was dissolved in 2ℓ of methanol, and after addition of 200 ml of concentrated hydrochloric acid, the solution was cooled to 0°C. While this solution was stirred, a saturated aqueous solution of 1.38 g (0.2 mol) of sodium nitrite was added dropwise at a temperature not exceeding 10°C. After completion of the reaction, 300 g of methyl acrylate was added and 10 g of copper(I) oxide was added gradually. The mixture was allowed to stand at room temperature overnight. After the evolution of nitrogen gas had terminated, the reaction mixture was concentrated to dryness under reduced pressure. The residue was diluted with water (1ℓ) - ether (2ℓ) and the ether layer was separated and dried over anhydrous magnesium sulfate. The ether was then distilled off and the residue was dissolved in 200 ml of ethanol. To this solution were added 15.2 g (0.2 mol) of thiourea and 16.5 g (0.2 mol) of sodium acetate and the mixture was stirred on an oil bath at 140°C for 15 hours. Then, 100 ml of 4N-hydrochloric acid was added and the mixture was stirred on an oil bath at 140°C for 15 hours. The ethanol was then distilled off under reduced pressure and the residue was diluted with 500 ml of water and 500 ml of ethyl acetate. The ethyl acetate layer was separated and dried over anhydrous magnesium sulfate and the ethyl acetate was distilled off. The residue was subjected to silica gel column chromatography (eluent: chloroform containing 2% of methanol) and the eluate corresponding to Rf=0.14 was pooled to give 11 g of 1,7-bis[4-[(2,4-dioxo-5-thiazolidinyl)methyl]phenoxy]heptane, m.p. 147°C. Byproducts were detected at Rf=0.61 and Rf=0.44.

Example 15

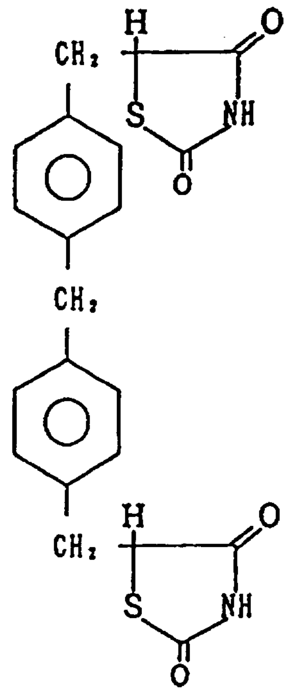

To a mixture of 300 ml of concentrated hydrochloric acid and 500 ml of methanol was added 4,4-diaminodiphenylmethane (99 g, 0.5 mol) for conversion to the hydrochloride and an aqueous solution of sodium nitrite (69 g (1 mol) of sodium nitrate in 200 ml of water) was added dropwise at 0-10 ° C, with constant stirring under ice-cooling. To this mixture was added 516 g (6 mols) of methyl acrylate and, then, 10 g of copper(I) oxide was added gradually. After the production of foam began, reaction mixture was allowed to stand at room temperature overnight. The methanol and excess methyl acrylate were then distilled off under reduced pressure and the residue was extracted with 700 ml of ether. The ether was then distilled off and the residue was dissolved in 500 ml of ethanol. To this solution were added 83.6 g (1.1 mols) of thiourea and 90.2 g (1.1 mols) of sodium acetate, and the mixture was refluxed with stirring for 3 days. The crystallized precipitate was collected by filtration and washed with ethanol. To this precipitate were added 500 ml of 4N-hydrochloric acid and 300 ml of EtOH and the mixture was refluxed with stirring for 3 days. The ethanol was then distilled off and the residue was dissolved in 500 ml of ether. The solution was washed with water and the ether was distilled off. The residue was subjected to silica gel column chromatography and the relevant eluate was pooled and recrystallized from ethyl acetate to give 35 g of 4,4'-bis[(2,4-dioxo-5-thiazolidinyl)methyl]diphenylmethane.

The following compounds were obtained in substantially the same manner as described above.

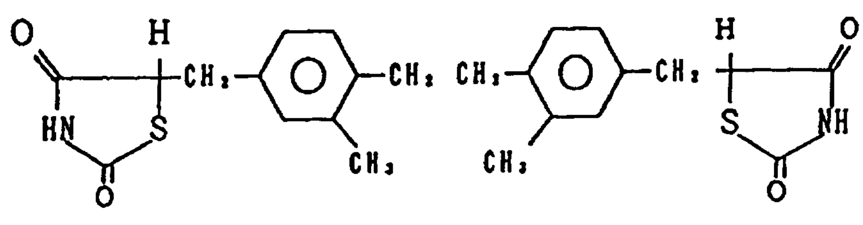

4,4'-Bis[(2,4-dioxo-5-thiazolidinyl)methyl-2,2'-dimethyl]bibenzyl
Starting compound: 4,4'-Diamino-2,2'-dimethylbibenzyl

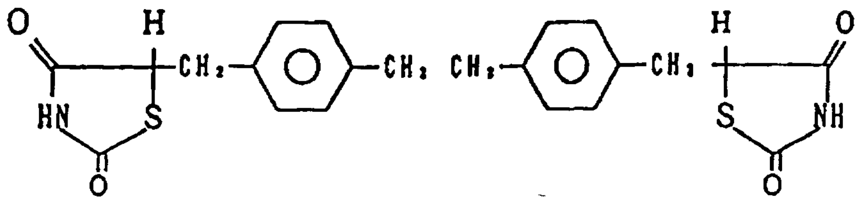

4,4-Bis[(2,4-dioxo-5-thiazolidinyl)methyl]bibenzyl
Starting compound: 4,4'-Diaminobibenzyl

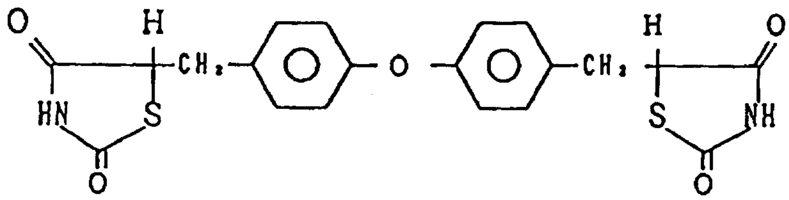

4,4-Bis[(2,4-dioxo-5-thiazolidinyl)methyl]diphenyl ether
Starting compound: 4,4'-Diaminodiphenyl ether

Example 16

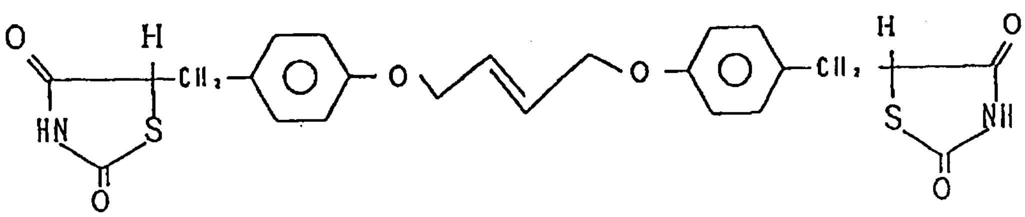

A mixture of 31 g (0.087 mol) of trans-1,4-bis[(p-acetylamino)phenyloxy]-2-butene and 40 ml of hydrochloric acid was refluxed for 12 hours. After cooling, a saturated aqueous solution of 12 g (0.174 mol) of sodium nitrite was added dropwise with ice-cooling and stirring for diazotization. Then, 100 ml of methyl acrylate was added and 5 g of copper(I) oxide was added with stirring. The mixture was then allowed to stand until the evolution of nitrogen gas had terminated. The excess methyl acrylate was distilled off and 200 ml of ether was added. The ether layer was separated, washed with water and dried over anhydrous magnesium sulfate. The ether was then distilled off and 13.1 g of thiourea, 14.1 g of sodium acetate and 100 ml of ethanol were added to the oily residue. The mixture was refluxed with stirring for 24 hours. Thereafter, 150 ml of 4N-hydrochloric acid was added and the mixture was refluxed for 12 hours with stirring. The ethanol was distilled off, followed by addition of 200 ml of ethyl acetate. The organic layer was separated, washed with water and concentrated to dryness under reduced pressure. The residue was subjected to silica gel column chromatography (eluent: chloroform-methanol = 50:1) and the relevant eluate was pooled and concentrated to dryness. Recrystallization from methanol gave trans-1,4-bis[p-[(2,4-dioxo-5-thiazolidinyl)methyl]phenoxy]-2-butene, m.p. 223-225 °C.

The following compound was obtained in substantially the same manner as above.

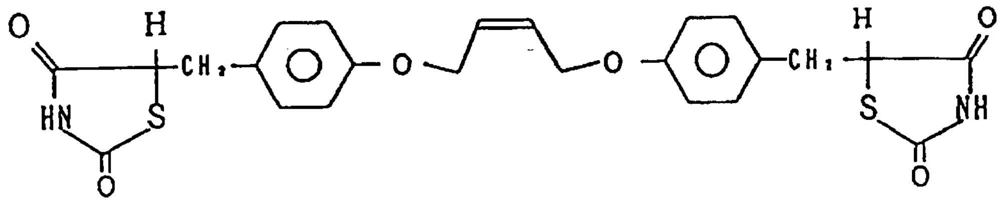

cis-1,4-Bis[p-[(2,4-dioxo-5-thiazolidinyl)methyl]phenoxy]-2-butene
Starting compound: cis-1,4-Bis[(p-acetylamino)phenoxy]-2-butene
The following compounds were also obtained in substantially the same manner.

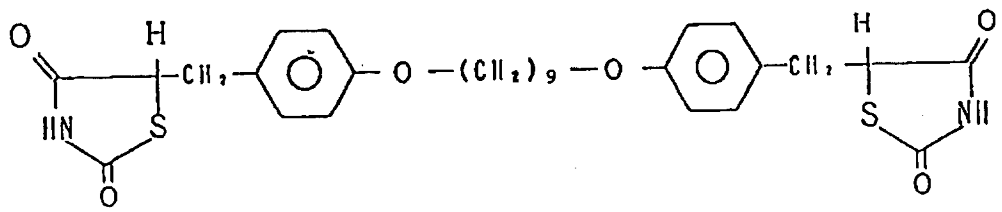

1,9-Bis[p-[(2,4-dioxo-5-thiazolidinyl)methyl]phenoxy]nonane
Starting compound: 1,9-Bis[p-acetylamino)phenoxy]nonane

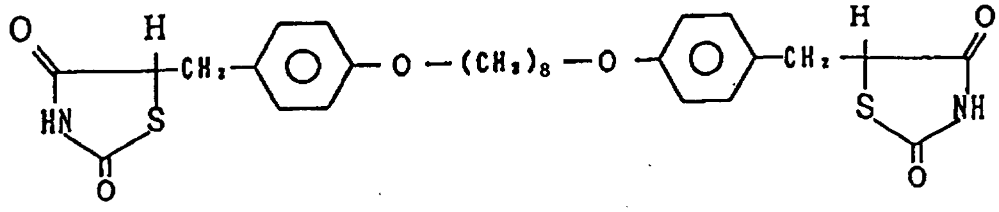

1,8-Bis[p-[(2,4-dioxo-5-thiazolidinyl)methyl]phenoxy]octane
Staring compound: 1,8-Bis[p-acetylamino)phenoxy]octane

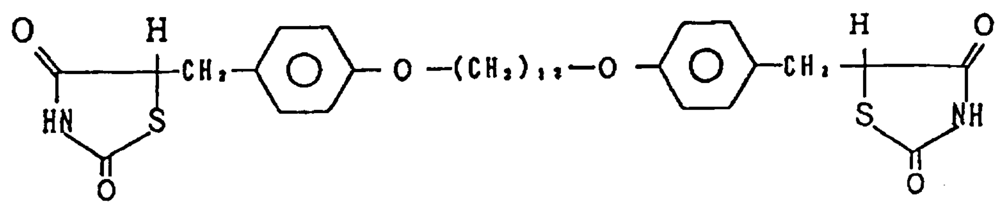

1,12-Bis[p-[(2,4-dioxo-5-thiazolidinyl)methyl]phenoxy]dodecane
Starting compound; 1,12-Bis[(p-acetylamino)phenoxy]dodecane

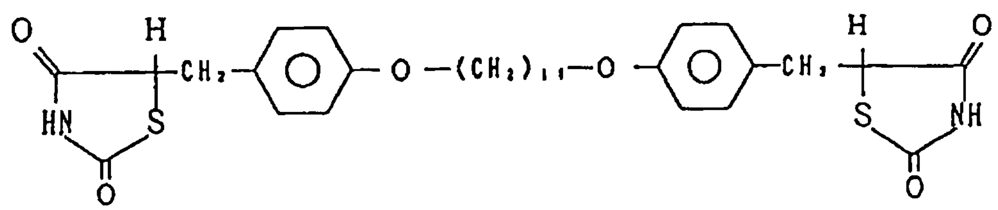

1,11-Bis[p-[(2,4-dioxo-5-thiazolidinyl)methyl]phenoxy]undecane
Starting compound; 1,11-Bis[(p-acetylamino)phenoxy]undecane

Example 17

To a solution of 4.0 g (0.0084 mol) of bis[p-[(2,4-dioxo-5-thiazolidinyl)methyl]phenyl] disulfide in dioxane (34 ml)-pure water (8.5 ml) was added 2.2 g (0.0084 mol) of triphenylphosphine, followed by addition of 1 drop of concentrated hydrochloric acid and the mixture was stirred at 40 ° C for 15 minutes. After completion of the reaction, the solvent was distilled off under reduced pressure and the residue was dissolved in benzene and dried over anhydrous magnesium sulfate. The solvent was then distilled off and the residue was dissolved in dimethylformamide (68 ml). To this solution were added 4.3 g (0.0168 mol) of p-(4-bromobutyloxy)benzaldehyde and 1.16 g (0.0084 mol) of potassium carbonate and the mixture was stirred at room temperature for 15 hours. After completion of the reaction, 1N-hydrochloric acid was added and the mixture was extracted with ethyl acetate. The extract was washed with a saturated aqueous solution of sodium chloride and dried over anhydrous magnesium sulfate. The solvent was then distilled off and the residue was dissolved in acetic acid (10 ml), followed by addition of 0.82 g (0.0106 mol) of ammonium acetate and 2.0 g (0.0168 mol) of 2,4-thiazolidinedione. The mixture was refluxed for 14 hours and, then, cooled. The resulting crystals were collected by filtration and washed with chloroform to give 5.4 g (63%) of crude crystals. A 2.0 g portion of this crop of crystals was recrystallized from ethyl acetate to give 1.62 g of 5-[[p-[[4-[[p-[(2,4-dioxo-5-thiazolidinyl)methyl]phenyl]thio]butyl]oxy]phenyl]methylene]thiazolidine-2,4-dione, m.p. 180-182 ° C (ethyl acetate).

The following compound was obtained in substantially the same manner as above.

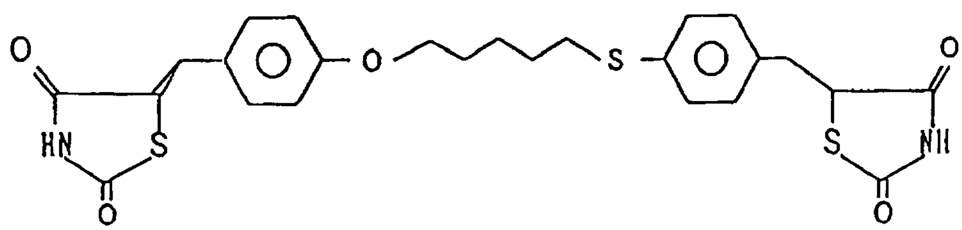

5-[[p-[[5-[[p-[(2,4-Dioxo-5-thiazolidinyl)methyl]phenyl]thio]pentyl]oxy]phenyl]methylene]thiazolidine-2,4-dione

Starting compound: p-(5-Bromopentyloxy)benzaldehyde

Example 18

To a solution of 4.0 g (0.0078 mol) of 5-[[p-[[4-[(2,4-dioxo-5-thiazolidinyl)methyl]phenyl]thio]butyl]oxy]-phenyl]methylene]thiazolidine-2,4-dione in dimethylimidazolidione (20 ml) was added 2.06 g (0.0545 mol) of sodium borohydride with ice-cooling. The mixture was stirred at room temperature for 1 hour and, then, at 80 ° C for 13 hours. The reaction mixture was cooled and poured in about 200 ml of ice-water and acidified with concentrated hydrochloric acid. This mixture was extracted with chloroform and the extract was washed with water and a saturated aqueous solution of sodium chloride and dried over anhydrous magnesium sulfate. The solvent was distilled off and the residue was purified by silica gel column chromatography (r-hexane-ethyl acetate = 2:1) and recrystallized from methanol to give 0.73 g (19%) of 5-[[p-[[-4-[[p-[(2,4-dioxo-5-thiazolidinyl)methyl]phenyl]thio]butyl]oxy]phenyl]methyl]thiazolidine-2,4-dione, m.p. 221-223 ° C (methanol).

The following compound was obtained in substantially the same manner as above.

5-[[p-[[5-[[p-[(2,4-Dioxo-5-thiazolidinyl)methyl]phenyl]thio]pentyl]oxy]phenyl]methyl]thiazolidine-2,4-dione

Starting compound: 5-[[p-[[5-[[p-[(2,4-Dioxo-5-thia zolidinyl)methyl]phenyl]thio]pentyl]oxy]phenyl]-methylene]thiazolidine-2,4-dione Melting point 73-75 ° C

Example 19

To a suspension of 0.62 g (0.0012 mol) of 1,4-bis[[p-[(2,4-dioxo-5-thiazolidinyl)methyl]phenyl]thio] butane in methylene chloride (15 ml) was added 1.1 g (80%; 0.0051 mol) of m-chloroperbenzoic acid with ice-cooling. The mixture was stirred for 30 minutes, at the end of which time 0.1 g (80%, 0.0005 mol) of m-chloroperbenzoic acid was further added and the mixture stirred for another 30 minutes. After completion of the reaction, a 10% aqueous solution (15 ml) of sodium thiosulfate was added and the insoluble matter was collected by filtration and washed with methanol. The resulting crude crystals were washed with hot

methanol to give 0.57 g (82%) of 1,4-bis[[p-[(2,4-dioxo-5-thiazolidinyl)methyl]phenyl]sulfonyl]butane, m.p. 235-240 ° C.

The following compound was obtained in substantially the same manner as above.

5-[[p-[[4-[[p-[(2,4-Dioxo-5-thiazolidinyl)methyl]phenyl]sulfonyl]butyl]oxy]phenyl]methyl]thiazolidine-2,4-dione

Starting compound: 5-[[p-[[4-[[p-[(2,4-Dioxo-5-thiazolidinyl)methyl]phenyl]thio]butyl]oxy]phenyl]methyl]thiazolidine-2,4-dione

Melting point 143-145 ° C

Example 20

A mixture of 3.34 g of 1,5-bis[(4-cyanomethyl)phenyloxy]pentane, 2.78 g of hydroxylamine hydrochloride, 7.71 g of sodium methoxide (28% in methanol) and 100 ml of methanol was stirred at room temperature for 3 hours and, then, refluxed for 24 hours. The reaction mixture was concentrated under reduced pressure and ethyl acetate and water were added to the residue. The resulting crystals were collected by filtration and dissolved in dichloromethane (150 ml)-pyridine (7.3 ml). Then, a solution consisting of 1.58 ml of thionyl chloride and 2 ml of dichloromethane was added dropwise with ice-cooling. The mixture was further stirred for 1 hour and, then, diluted with water. The organic layer was separated and dried over magnesium sulfate. The solvent was distilled off under reduced pressure and the residue was subjected to silica gel column chromatography (10% methanol in chloroform) to give 0.98 g of 1,5-bis[-[p-(2-oxo-3H-1,2,3,5-oxathiazol-4-yl)methyl]phenyloxy]propane, m.p. 134-135 ° C.

Example 21

A mixture of 5.14 g of 1,5-bis[p-[(2,4-dioxo-5-thiazolidinyl)methyl]phenoxy]propane, 2.80 g of potassium carbonate, 3.7 ml of methyl iodide and 50 ml of dimethylformamide was stirred at 60 ° C for 2 hours. The reaction mixture was then diluted with water and ethyl acetate and the organic layer was separated, washed with a saturated aqueous solution of sodium chloride and dried over anhydrous magnesium sulfate. The solvent was then distilled off under reduced pressure and the residue was purified by silica gel column chromatography (hexane-ethyl acetate = 2:15) to give 3.0 g of 1,5-bis[p-[(2,4-dioxo-3-methyl-5-thiazolidinyl)methyl]phenoxy]propane, m.p. 101-102 ° C (ether).

Reference Example 2

To a solution of 11.2 g (0.050 mol) of 5-(p-hydroxybenzyl)thiazolidine-2,4-dione in dimethylformamide (125 ml) was added 4.0 g (60%, 0.100 mol) of sodium hydride with ice-cooling, and the mixture was stirred at room temperature for 30 minutes. Then, a solution of 13.6 g (0.050 mol) of p-(5-bromopentyloxy)-benzaldehyde in dimethylformamide (50 ml) was added dropwise and the mixture was stirred at room temperature for 1.5 hours and, then, at 75 ° C for 3 hours. The reaction mixture was poured in ice-cooled 4N-hydrochloric acid and extracted with ethyl acetate. The extract was washed with a saturated aqueous solution of sodium chloride and dried over anhydrous magnesium sulfate. The solvent was then distilled off and the residue was purified by silica gel column chromatography (chloroform) to give 6.2 g (30%) of p-[[5-[[p-[(2,4-dioxo-5-thiazolidinyl)methyl]phenyl]oxy]pentyl]oxy]benzaldehyde as crude crystals.

A portion of the above crop of crystals was recrystallized from methanol. Melting point 103-106 ° C (methanol).

Reference Example 3

A mixture of 31.2 g (0.1 mol) of 1,5-bis(4-formylphenoxy)pentane, 11.7 g (0.1 mol) of thiazoline-2,4-dione, 3 g of ammonium acetate and 200 ml of acetic acid was stirred with heating at 70-100 ° C for 12 hours. The resulting crystals were collected by filtration and washed with acetic acid and methanol in that order to give 23 g of 5-(4-formylphenoxy)-1-[4-[(2,4-dioxo-5-thiazolidinylidene)methyl]phenoxy]pentane, m.p. 230-240 ° C.

Example 22

A mixture of 17.76 g of 1,4-bis(p-formylphenoxy)-trans-2-butene, 15.44 g of 2,4-thiazolidinedione, 2.31 g of ammonium acetate and 200 ml of acetic acid was refluxed for 3 days and the resulting crystals were

collected by filtration. The crystals (7.7 g) were suspended in 40 ml of dimethylimidazolidinone followed by addition of 2.7 g of sodium borohydride, and the mixture was stirred at 80°C for 2 hours. The reaction mixture was added to a mixture of 23 ml of concentrated hydrochloric acid, 200 ml of ice-water and 200 ml of ethyl acetate and the organic layer was separated, washed with water and dried over magnesium sulfate. The solvent was distilled off and the residue was purified by silica gel column chromatography (toluene-ethyl acetate = 3:1) to give 1.53 g of 1,4-bis[p-[(2,4-dioxothiazolidinyl)methyl]phenoxy]-trans-2-butene.

The physicochemical properties of this compound were in agreement with those of the product obtained in Example 16.

The following compound was also obtained in substantially the same manner.

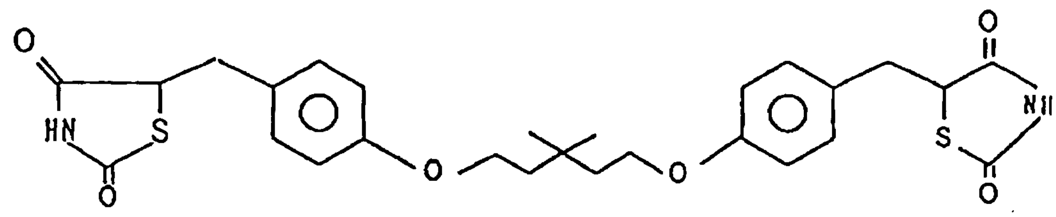

3,3-Dimethyl-1,5-bis[p-[(2,4-dioxo-5-thiazolidinyl)methyl]phenoxy]pentane (m.p. 141-142°C)
Starting compound: 3,3-Dimethyl-1,5-bis(p-formylphenoxy)pentane

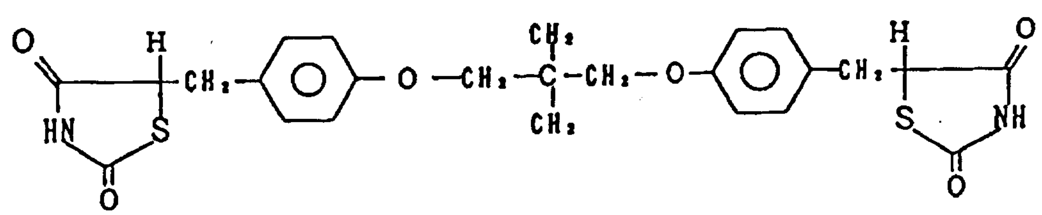

1,3-Bis[p-[(2,4-dioxo-5-thiazolidinyl)methyl]phenoxy]-2,2-dimethylpropane
Starting compound: 1,3-Bis[p-formylphenoxy]-2,2-dimethylpropane

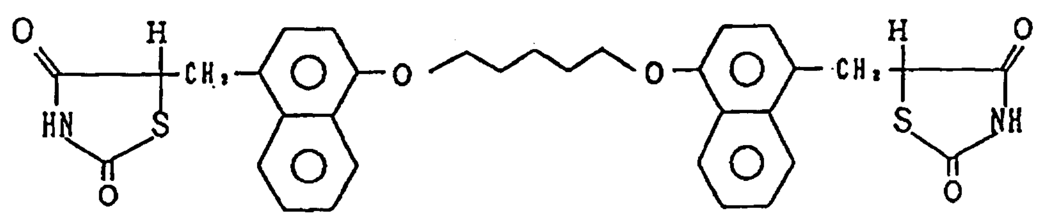

1,5-Bis[4-[(2,4-dioxo-5-thiazolidinyl)methyl]naphthoxy]pentane
Starting compound: 1,5-Bis[p-formylnaphthoxy]pentane

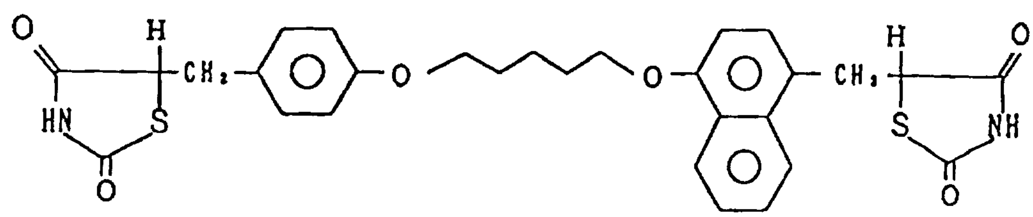

1-[4-[(2,4-Dioxo-5-thiazolidinyl)methyl]naphthoxy]-5-[4-[(2,4-dioxo-5-thiazolidinyl)methyl]phenoxy]-pentane (resinous)
Starting compound: 1-(4-Formylnaphthoxy)-5-(4-formylphenoxy)pentane

Example 23

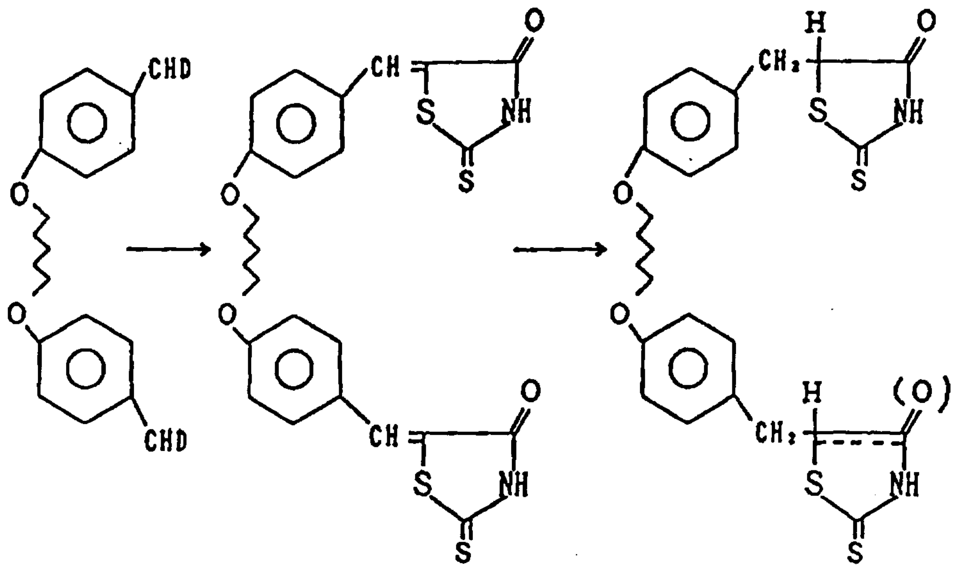

A mixture of 9.36 g (0.03 mol) of 1,5-bis(4-formylphenoxy)pentane, 9.36 g (0.07 mol) of rhodanine, 3 g of ammonium acetate and 200 ml of acetic acid was refluxed with stirring for 72 hours. The crystallized precipitate was collected by filtration when hot and washed with acetic acid and methanol in that order to give 1,5-bis[4-[(5-rhodanylidene)methyl]phenoxy]pentane, m.p. 285-290 ° C. This yellow precipitate (5.42 g) was suspended in dimethylimidazolidinone followed by addition of 6 g of sodium borohydride. The mixture was heated at 70-80 ° C for 20 hours, after which it was dispersed in 300 ml of water and extracted with 500 ml of ethyl acetate. The ethyl acetate was distilled off and the residue was subjected to silica gel chromatography. The eluate corresponding to Rf = 0.9 and those corresponding to Rf = 0.4 (developing solvent chloroform-ethyl acetate = 3:1) were respectively pooled. The eluate of Rf = 0.9 was treated to give 1.5 g of 1,5-bis[4-[(5-rhodanyl)methylphenoxy]pentane. The eluate of Rf = 0.4 was also treated to give 0.3 g of 1-[4-[(5-rhodanyl)methyl]phenoxy]-5-[4-(2-thioxo-4-thiazolin-5-yl)methyl]phenoxy]pentane.

Example 24

A mixture of 4.11 g (0.01 mol) of the 1-[4-[(2,4-dioxo-5-thiazolididene)methyl]phenoxy]-5-(4-formylphenoxy)pentane obtained in Reference Example 3, 1 g of ammonium acetate, 1.37 g (0.01 mol) of rhodanine and 300 ml of acetic acid was refluxed with stirring for 72 hours. The insoluble precipitate was recovered by filtration and recrystallized from dimethylformamide to give 2.6 g of 1-[4-[(2,4-dioxo-5-thiazolididene)methyl]phenoxy]-5-[4-(5-rhodanylidene)methyl]phenoxy]pentane, m.p. ≧310 ° C. This precipitate was suspended in 10 ml of dimethylimidazolidinone followed by addition of 2.5 g of sodium borohydride, and the mixture was heated at 60-70 ° C for 12 hours. The reaction mixture was dispersed in 100 ml of water and extracted with 200 ml of ethyl acetate. The ethyl acetate was distilled off and the residue was subjected to silica gel column chromatography (eluent: chloroform-ethyl acetate = 2:1). The relevant eluate was pooled to give 0.7 g of 1-[4-[(2,4-dioxo-5-thiazolidinyl)methyl]phenoxy]-5-[4-(5-rhodanyl)-methyl]phenoxy]pentane.

Example 25

A mixture of 413 mg (0.001 mol) of the 5-(4-formylphenoxy)-1-[4-[(2,4-dioxo-5-thiazolidinyl)methyl]-phenoxy]pentane obtained in Reference Example 3, 137 mg (0.001 mol) of rhodanine, 0.1 g of ammonium acetate and 5 ml of acetic acid was refluxed for 24 hours and cooled off. The resulting crystals were collected by filtration and recrystallized from acetic acid to give 250 mg of 1-[4-[(2,4-dioxo-5-thiazolidinyl)-methyl]phenoxy]-5-[4-[(5-rhodanylidene)methyl]phenoxy]pentane.

The following compounds were obtained in substantially the same manner as above.

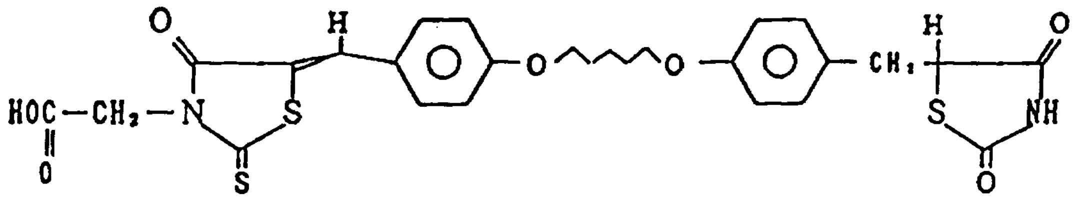

1-[4-[(2,4-Dioxo-5-thiazolidinyl)methyl]phenoxy]-5-[4-[[N-(hydroxycarbonylmethyl)-5-rhodanylidene)-methyl]phenoxy]pentane.

Starting compound: Rhodanine-3-acetic acid (the product obtained in Reference Example 2)

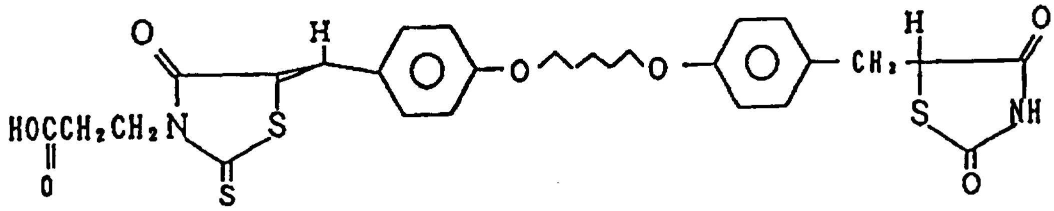

1-[4-[(2,4-Dioxo-5-thiazolidinyl)methyl]phenoxy]-5-[4-N-[2-hydroxycarbonyl)ethyl)-5-rhodanylidene)-methyl]phenoxy]pentane.

Starting compound: Rhodanine-3-propionic acid

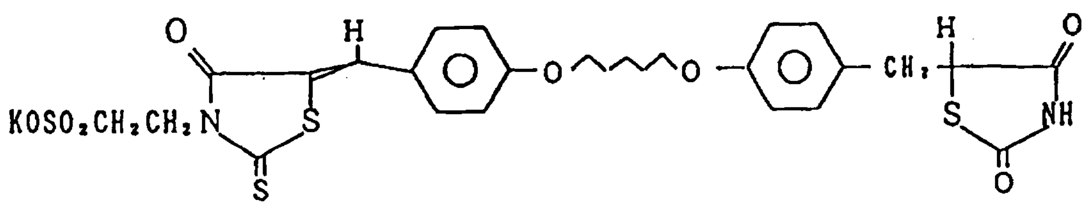

1-[4-[(2,4-Dioxo-5-thiazolidinyl)methyl]phenoxy]-5-[4-[N-[2-(hydroxysulfonyl)ethyl-5-rhodanylidene]-methyl]phenoxy]pentane potassium

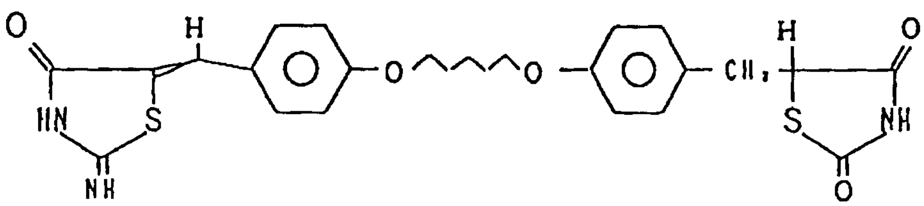

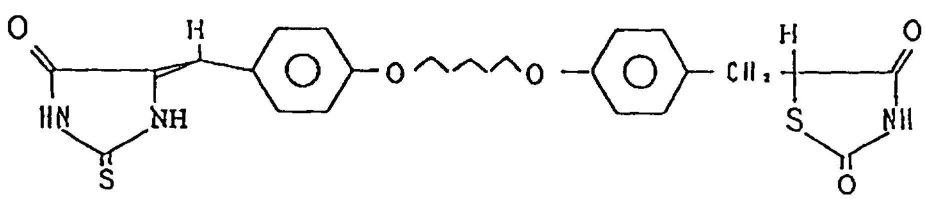

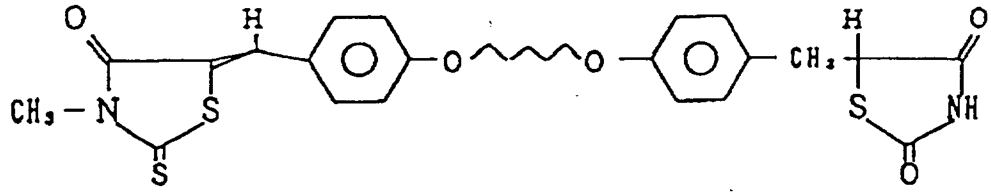

1-[4-[(2,4-Dioxo-5-thiazolidinyl)methyl]phenoxy]5-[4-[N-methyl-5-rhodanylidene)methyl]phenoxy]pentane
Starting compound: N-Methylrhodanine

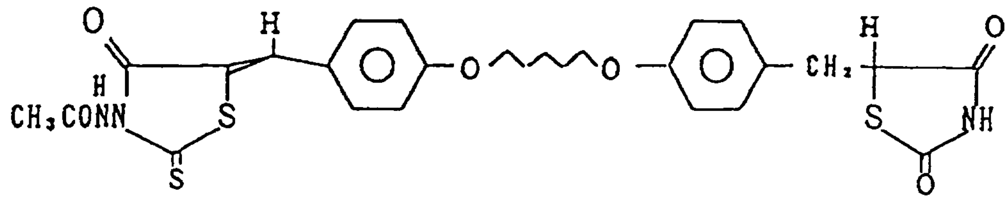

1-[4-[(2,4-Dioxo-5-thiazolidinyl)methyl]phenoxy]5-[N-(acetylamino)-5-rhodanylidene]methyl]phenoxy]-pentane
Starting compound: N-Aminorhodanine

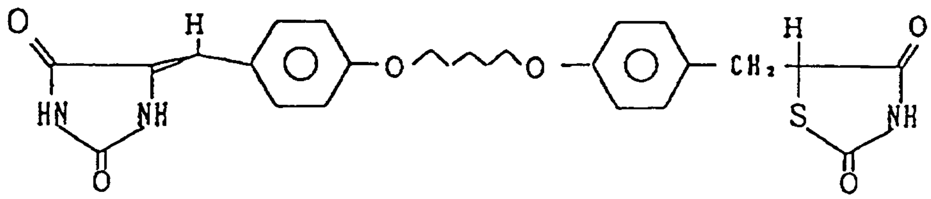

1-[4-[(2,4-Dioxo-5-thiazolidinyl)methyl]phenoxy]-5-[4-[2,4-dioxo-5-imidazolinylidene]methyl]phenoxy]-pentane

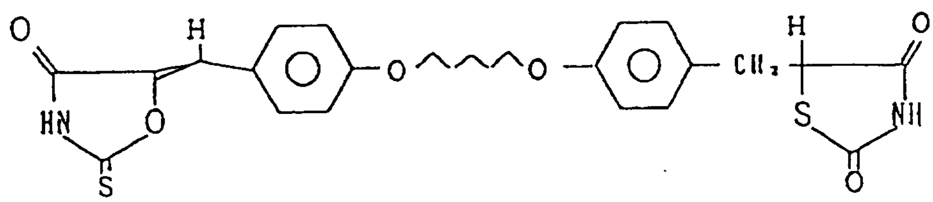

1-[4-[(2,4-Dioxo-5-thiazolidinyl)methyl]phenoxy]-5-[4-[(4-oxo-2-thioxo-5-oxazolidinylidene)methyl]-phenoxy]pentane
Starting compound: 4-Oxo-2-thioxooxazolidine

Example 26

To a solution of 18.37 g (0.039 mol) of bis(4-(2,4-dioxo-5-thiazolidinyl)methyl]phenyl] disulfide in ethanol (180 ml) was added 2.92 g (0.077 mol) of sodium borohydride and the mixture was stirred at room temperature for 2 hours. After completion of the reaction, water and 1N-hydrochloric acid were added and the mixture was extracted with ether. The extract was washed with a saturated aqueous solution of sodium chloride and dried over anhydrous magnesium sulfate. The solvent was then distilled off and the residue was purified by column chromatography (chloroform) and further by column chromatography (n-hexane-ethyl acetate = 4:1) to give 3.05 g (16%) of 5-(4-mercaptobenzyl)thiazolidine-2,4-dione, m.p. 84.5-85 ° C.

Example 27

To 20 ml of dimethylformamide were added 2.4 g (0.01 mol) of 5-(4-mercaptobenzyl)thiazolidine-2,4-dione, 0.7 g (0.005 mol) of anhydrous potassium carbonate and 1,5-dibromopentane (0.005 mol) and the mixture was stirred at room temperature for 15 hours.

After completion of the reaction, 100 ml of water and 100 ml of ethyl acetate were added and the ethyl acetate layer was separated and washed with water. The solvent was then distilled off and the residue was subjected to silica gel column chromatography (eluent: chloroform). The relevant eluate was pooled and concentrated to dryness to give 0.5 g of crystals. The physicochemical properties of this product were in agreement with those of 1,5-bis[[p-(2,4-dioxo-5-thiazolidinyl)methyl]phenyl]thio]pentane.

The reaction procedure of the present invention gave the following compounds as well.

Example 28

1-[[4-(2,4-Dioxo-5-thiazolidinylidene]methyl]naphthoxy]-5-[(4-(2,4-dioxo-5-thiazolidinylidene)methyl]-phenoxy]pentane (m.p.: 227-230 ° C)

Starting compound: 2,4-Dioxo-5-[(4-hydroxynaphthyl)methylidene]thiazolidine

Example 29

1,5-Bis[4-[(2,4-dioxo-5-imidazolidinylidene)methyl]phenoxy]butane(m.p.: 285-288 ° C)

Starting compound: 1,5-(4-Formylphenoxy)pentane

Example 30

1,5-Bis[(2,4-Dioxo-5-thiazolidinyl)methyl]naphthalene (m.p.: ≧300 ° C)

Starting compound: 1,5-Diaminonaphthalene

Example 31

1,4-Bis[4-[(4-oxo-2-thioxo-5-oxazolidinylidene)methyl]phenoxy]pentane (m.p.: 233-235 ° C)

Starting compound: 1,5-Bis(4-formylphenoxy)pentane

Example 32

1,4-Bis[[4-(2,4-dioxo-5-thiazolidinylidene)methyl]phenoxy]-2-butene (m.p.: 290-295 ° C)

Starting compound: 1,4-Bis(4-formylphenoxy)-2-butene

The following compounds were also produced.

Example 33

1,7-Bis[3-[(4-oxo-2-thioxo-5-thiazolidinylidene)methyl]phenoxy]heptane (m.p.: 173-180 ° C) (acetic acid)

Example 34

1,7-Bis[2-[(2,4-dioxo-5-thiazolidinylidene)methyl]phenoxy]heptane(m.p.: 224-226 ° ) (acetic acid)

Example 35

1,7-Bis[4-[(2,4-dioxo-5-thiazolidinylidene)methyl]phenoxy]heptane(m.p.: 213-218 ° C) (acetic acid)

Example 36

1,7-Bis[3-[(5-rhodanylidene)methyl]phenoxy]heptane (m.p.: 238-239 ° C)

Example 37

1,5-Bis[4-[(4-oxo-2-thioxo-5-oxazolidinylidene)methyl]phenoxy]heptane (m.p.: 233-235 ° C) (acetic acid)

Example 38

In 300 ml of N,N'-dimethylimidazolidinone was dissolved 2.55 g of 1,5-bis[4-[(4-oxo-2-thioxo-5-oxazolidinylidene)methyl]phenoxy]pentane with heating and the mixture was stirred under ice-cooling. When the internal temperature had reached 20°C, 2.55 g of m-chlorobenzoic acid was added. The mixture was further stirred at room temperature for 18 hours, at the end of which time 300 ml of water was added. The mixture was further stirred for 2 hours and the crystallized precipitate was recovered by filtration, washed with a small amount of N,N'-dimethylformimidazolidinone and washed thoroughly with methanol. The resulting crystals were purified by reprecipitation from DMF and water to give 1,5-bis[4-[(2,4-dioxo-5-oxazolidinylidene)methyl]phenoxy]pentane, m.p. 266-267°C (dimethylformamide + water).

Example 39

In 100 ml of tetrahydrofuran was dissolved 1.0 g of 1,5-bis[4-[(2,4-dioxo-5-oxazolidinylidene)methyl]-phenoxy]pentane. Then, 2 g of 10% palladium-on-carbon was added and the mixture was stirred in a hydrogen gas atmosphere until no more hydrogen was absorbed. The palladium-on-carbon was filtered off and the solvent was distilled off under reduced pressure. Finally, the residue was purified by silica gel column chromatography (chloroform-methanol = 10:1) to give 1,5-[4-[(2,4-dioxo-5-oxazolidinyl)methyl]-phenoxy]pentane.

Example 40

A mixture of 700 mg of 1,5-bis[p-(2-ethoxycarbonyl-2-hydroxyethyl)phenoxy]pentane, 310 mg of urea, 0.77 ml of 28% sodium methylate and 15 ml of ethanol was stirred at room temperature for 1 hour and, then, refluxed for 3 hours. The reaction mixture was concentrated and 2N-hydrochloric acid was added to the residue, followed by extraction with ethyl acetate. The extract was washed with a saturated aqueous solution of sodium chloride and dried over anhydrous magnesium sulfate, and the solvent was distilled off. The residue was purified by silica gel column chromatography (hexane-ethyl acetate = 1:2) to give 210 mg of a compound identical with the compound obtained in Example 39 (m.p. 112-113°C).

| Example | Melting point | Elemental analysis C(%) | H(%) | N(%) | S(%) | Mass spectrum (m/z) | NMR spectrum ($d_6$-DMSO, TMS internal standard) |
|---|---|---|---|---|---|---|---|
| | | Calcd. | | | | | |
| | | Found | | | | | |
| 14 | 147°C | ($C_{27}H_{30}N_2O_6S_2$) | | | | | |
| | (EtOH) | 59.76 | 5.57 | 5.16 | 11.82 | 541(M-1)$^-$ | 1.2-1.9; 2.9-3.4; |
| | | 59.53 | 5.61 | 4.96 | 11.83 | | 3.93; 4.9; 6.8-7.2; |
| | | | | | | | 12.0 |
| 15 | 214.4°C | ($C_{21}H_{18}N_2O_4S_2$) | | | | | |
| | (EtOH) | 59.14 | 4.25 | 6.57 | 15.04 | | |
| | | 59.16 | 4.30 | 6.52 | 15.15 | 425(M-1)$^-$ | 3.06; 3.35; 3.90; |
| | | | | | | | 4.89; 7.17; 12.0 |
| | 240-1°C | ($C_{24}H_{24}N_2O_4S_2$) | | | | | |
| | (Iso-PrOH) | 61.52 | 5.16 | 5.98 | 13.69 | | 2.19; 2.74; 3.03; |
| | | 61.47 | 5.11 | 5.71 | 13.43 | 467(M-1)$^-$ | 3.35; 4.87; 7.02; |
| | | | | | | | 12.02 |

| | | | | | | | |
|---|---|---|---|---|---|---|---|
| | 232-2°C | ($C_{22}H_{20}N_2O_4S_2$) | | | | | |
| | (Iso-PrOH) | 59.98 | 4.58 | 6.36 | 14.56 | | |
| | | 60.08 | 4.64 | 6.12 | 14.40 | 439(M-1)$^-$ | 3.07; 3.31; 4.90; |
| | | | | | | | 7.14; 12.01 |
| | 178-180°C | ($C_{20}H_{16}N_2O_5S_2$) | | | | | |
| | (AcOEt) | 56.06 | 3.76 | 6.54 | 14.97 | | 3.12; 3.35; 4.90; |
| | | 56.07 | 3.81 | 6.34 | 14.88 | 429(M-1)$^-$ | 6.92; 7.26; 12.03 |
| 16 | 223-5°C | ($C_{24}H_{22}N_2O_6S_2$) | | | | | 3.05; 3.35; 4.60; |
| | | 57.82 | 4.45 | 5.62 | 12.86 | 497(M-1)$^-$ | 4.89; 6.06; |
| | | 57.63 | 4.38 | 5.43 | 12.59 | | 6.88-7.10; 11.97 |
| | | ($C_{24}H_{22}N_2O_6S_2$) | | | | | |
| | | 57.82 | 4.45 | 5.62 | 12.86 | 497(M-1)$^-$ | 2.9-3.6; 4.76; 4.92 |
| | | 57.56 | 4.63 | 5.57 | 12.25 | | 5.92; 6.92-7.28; |
| | | | | | | | 10.5-13.0 |

| | | | | | | |
|---|---|---|---|---|---|---|
| 115-7°C | ($C_{29}H_{34}N_2O_6S_2$) | | | | | |
| (MeOH) | 61.03 | 6.00 | 4.91 | 11.24 | 569(M-1)$^-$ | 1.2-1.9; 3.04; |
| | 60.82 | 5.95 | 4.74 | 11.30 | | 3.24; 3.92; 6.85; |
| | | | | | | 7.14; 11.99 |
| | ($C_{28}H_{32}N_2O_6S_2$) | | | | | |
| | 60.41 | 5.79 | 5.03 | 11.52 | | |
| | 60.38 | 5.80 | 4.85 | 11.42 | 555(M-1)$^-$ | 1.2-1.8; 3.06; 3.35; |
| | | | | | | 4.86; 6.87; 7.18; 12.0 |
| 145-8°C | ($C_{32}H_{40}N_2O_6S_2$) | | | | | 1.2-1.76; 3.06; 3.3; |
| (MeOH) | 62.72 | 6.58 | 4.57 | 10.47 | | 3.92; 4.86; 6.87; 7.15; |
| | 62.50 | 6.58 | 4.39 | 10.21 | 611(M-1)$^-$ | 12.0 |
| | ($C_{31}H_{38}N_2O_6S_2$) | | | | | 1.2-1.77; 3.06; 3.34; |
| | 62.18 | 6.40 | 4.68 | 10.71 | | 3.92; 4.87; 6.87; 7.15; |
| | 61.88 | 6.38 | 4.54 | 10.69 | 597(M-1)$^-$ | 12.0 |

| | | | | | | | |
|---|---|---|---|---|---|---|---|
| 17 | 180-182°C (AcOEt) | ($C_{24}H_{22}N_2O_5S_3$) | | | | | |
| | | 56.01 | 4.31 | 5.44 | 18.69 | $513(M-1)^-$ | 1.6-1.9; 3.02; 3.07; |
| | | 55.93 | 4.39 | 5.38 | 18.31 | | 4.07; 4.90; 7.19; 7.29 |
| | | | | | | | 7.38; 7.56; 7.77; 12.03; |
| | | | | | | | 12.50 |
| | 119-121°C (AcOEt) | ($C_{25}H_{24}N_2O_5S_3$) | | | | $527(M-1)^-$ | 1.5-1.8; 2.97; 3.05; |
| | | 56.80 | 4.58 | 5.30 | 18.20 | | 4.02; 4.89; 7.08; |
| | | 56.78 | 4.48 | 5.13 | 17.99 | | 7.17; 7.27; 7.56; 7.76; |
| | | | | | | | 12.07; 12.43 |
| 18 | 221-223°C (MeOH) | ($C_{24}H_{24}N_2O_5S_3$) | | | | | |
| | | 55.79 | 4.68 | 5.42 | 18.62 | | |
| | | 55.75 | 4.64 | 5.33 | 18.74 | $517(M+1)^+$ | 1.7-1.9; 3.01; 3.95 |
| | | | | | | | 4.85-4.92; 6.86; |
| | | | | | | | 7.1-7.2; 7.26; 12.01 |

| | | | | | | | |
|---|---|---|---|---|---|---|---|
| | 73-75°C (MeOH) | ($C_{25}H_{26}N_2O_5S_3$) | | | | $531(M+1)^+$ | 1.5-1.7; 2.97; 3.00-3.12; 3.91; 4.85-4.91; 6.87; 7.1-7.2; 7.29; 12.02 |
| | | 56.58 | 4.94 | 5.28 | 18.13 | | |
| | | 56.32 | 4.95 | 5.17 | 18.19 | | |
| 19 | 235-40°C | ($C_{24}H_{24}N_2O_8S_4$) | | | | $595(M-1)^-$ | 1.60; 3.50; 5.00; 7.55; 7.80; 12.10 |
| | | 48.31 | 4.05 | 4.69 | 21.49 | | |
| | | 47.58 | 3.95 | 4.54 | 22.29 | | |
| | 143-5°C (MeOH) | ($C_{24}H_{24}N_2O_7S_3$) | | | | | |
| | | 52.54 | 4.41 | 5.11 | 17.53 | $549(M+1)^+$ | 1.7-1.8; 3.05; 3.38; 3.50; 3.90; 4.86; 4.99; 6.82; 7.13; 7.54; 7.85; 12.05 |
| | | 52.25 | 4.47 | 4.97 | 17.02 | | |
| 20 | 134-5°C ($CH_2Cl_2$) | | | | | $493(M+1)^+$ | 1.36-1.94; 3.74-4.10; 6.70-30 |

| | | | | | | | |
|---|---|---|---|---|---|---|---|
| 21 | 101-2°C | | | | | 541(M-1)$^-$ | 1.50-2.20; 2.80-3.70 |
| | | | | | | | 3.10; 3.80-4.20; |
| | | | | | | | 4.45; 6.70-7.30 |
| 22 | 141-2°C | ($C_{27}H_{30}N_2O_6S_2$) | | | | 541(M-1)$^-$ | 1.02; 1.73; 3.02-3.33 |
| | (MeOH) | 59.76 | 5.57 | 5.16 | 11.82 | | 4.02; 4.86; 6.87; 7.14; |
| | | 59.76 | 5.59 | 5.09 | 11.80 | | 12.00 |
| | 232-3°C | ($C_{25}H_{26}N_2O_6S_2$) | | | | 513(M-1)$^-$ | 1.09; 3.05; 3.29; |
| | (MeOH) | 58.35 | 5.09 | 5.44 | 12.46 | | 3.81; 4.85; 6.88; |
| | | 58.27 | 5.06 | 5.32 | 12.63 | | 7.13; 11.99 |
| | 129-31°C | ($C_{33}H_{30}N_2O_6S_2$) | | | | 613(M-1)$^-$ | 1.7-2.05; 3.41; 3.90; |
| | (EtOH) | 64.48 | 4.92 | 4.56 | 10.43 | | 4.94; 6.94; 7.31; |
| | | 64.57 | 5.04 | 4.43 | 10.32 | | 7.48; 7.60; 8.02; 8.24; |
| | | | | | | | 12.1 |

| | | | | | | | |
|---|---|---|---|---|---|---|---|
| | | $(C_{29}H_{28}N_2O_6S_2)$ | | | | | |
| | | 61.68 | 5.00 | 4.96 | 11.36 | $563(M-1)^-$ | 1.5-2.1; 3.11; 3.26; |
| | | 61.51 | 5.10 | 5.00 | 11.19 | | 3.4-3.5; 4.0; 4.17; |
| | | | | | | | 4.50; 4.69; 8.0; 8.13; |
| 23 | 117-122°C | $(C_{25}H_{26}N_2O_4S_4)$ | | | | | |
| | (EtOH) | 54.92 | 4.79 | 5.12 | 23.46 | $545(M-1)^-$ | 1.4-1.8; 3.1; 3.28; |
| | | 55.08 | 4.83 | 4.99 | 22.96 | | 3.95; 4.98; 6.86; |
| | | | | | | | 7.13; 13.1 |
| | | $(C_{25}H_{26}N_2O_3S_4)$ | | | | $529(M-1)^-$ | 1.5-1.8; 3.1; |
| | | 56.57 | 4.94 | 5.28 | 241.7 | | 3.3; 3.81; 3.95; |
| | | 56.52 | 5.11 | 5.15 | 23.49 | | 4.98; 6.7-7.2; |
| | | | | | | | 12.94; 13.12; |

| | | | | | | | |
|---|---|---|---|---|---|---|---|
| 25 | 170-200°C (AcOH) | ($C_{25}H_{24}N_2O_5S_3$) | | | | $527(M-1)^-$ | 1.4-1.9; 3.05; 3.30; 3.96; 4.07; 4.86; 7.61; 12.0; 13.74 |
| | | 56.80 | 4.58 | 5.30 | 18.20 | | |
| | | 56.73 | 5.01 | 4.92 | 17.31 | | |
| | 98-100°C (AcOH) | ($C_{27}H_{26}N_2O_7S_3$) | | | | $585(M-1)^-$ | 1.5-1.9; 3.05; 3.96; 4.10; 4.86; 7.86; 11.99; 13.6; |
| | | 55.27 | 4.47 | 4.77 | 16.40 | | |
| | | 54.99 | 4.51 | 4.60 | 16.25 | | |
| | 183-4°C (AcOH) | ($C_{28}H_{28}N_2O_7S_3$) | | | | $599(M-1)^-$ | 1.5-1.9; 2.63; 3.05; 3.30; 3.96; 4.09; 4.23; 4.86; 7.78; 11.99; 12.36 |
| | | 55.98 | 4.70 | 4.66 | 16.01 | | |
| | | 55.69 | 4.79 | 4.50 | 15.86 | | |

| | | | | | | |
|---|---|---|---|---|---|---|
| 235-237°C | ($C_{27}H_{27}N_2O_8S_4K$) | | | | 673(M-1)$^-$ | 1.5-1.9; 2.77; |
| (AcOH) | 48.05 | 4.03 | 4.15 | 19.01 | | 3.05; 3.96; 4.09; |
| | 48.54 | 4.61 | 5.40 | 19.11 | | 4.29; 4.86; 7.77; |
| | | | | | | 12.0; |
| 238-240°C | ($C_{25}H_{25}N_3O_5S_2$) | | | | 510(M-1)$^-$ | 1.4-2.0; 3.05; |
| (AcOH) | 58.69 | 4.93 | 8.21 | 12.54 | | 3.8-4.2; 4.86 |
| | 58.62 | 4.98 | 7.92 | 12.03 | | 9.0-9.7; 11.99 |
| 191-3°C | ($C_{25}H_{25}N_3O_5S_3$) | | | | 510(M-1)$^-$ | 1.5-1.9; 3.06; |
| (AcOH) | 58.69 | 4.93 | 8.21 | 12.54 | | 3.96; 4.06; 4.86; |
| | 58.10 | 4.98 | 8.06 | 12.16 | | 6.47; 12.04; |
| | | | | | | 12.29; |
| 163-5°C | ($C_{26}H_{26}N_2O_5S_3$) | | | | 541(M-1)$^-$ | 1.5-1.9; 3.05; |
| (AcOH) | 57.54 | 4.83 | 5.16 | 17.73 | | 3.29; 3.40; 3.96; |
| | 57.77 | 4.92 | 5.04 | 17.41 | | 4.09; 4.86; 7.79; |
| | | | | | | 11.99 |

| | | | | |
|---|---|---|---|---|
| | 96-9°C | ($C_{27}H_{27}N_3O_6S_3$)<br>55.37 4.65 7.17 16.42<br>54.96 4.68 7.12 16.42 | 584(M-1)$^-$ | 1.5-1.9; 2.08;<br>3.06; 3.97; 4.11;<br>4.87; 7.89; 11.15;<br>12.00; |
| | 220-221°C<br>(MeOH) | ($C_{25}H_{25}N_3O_6S$)<br>60.59 5.08 8.48 6.'47<br>60.68 5.16 8.44 6.49 | 494(M-1)$^-$ | 1.5-1.8; 3.05;<br>3.96; 4.03; 4.86;<br>6.38; 10.41; 11.15;<br>12.0 |
| | 101-3°C<br>(AcOH) | ($C_{25}H_{24}N_2O_6S_2$)<br>58.58 4.72 5.46 12.51<br>58.66 4.95 5.52 12.91 | 511(M-1)$^-$ | 1.5-1.9; 3.05;<br>3.29; 3.96; 4.07;<br>4.87; 6.77; 12.00;<br>13.80 |
| 26 | 84.5-85°C | ($C_{16}H_7NO_2S_2$)<br>50.19 3.79 5.85 26.80<br>50.38 3.87 5.62 26.58 | 239($M^+$) | 3.04; 3.31; 4.85;<br>5.33; 7.07; 7.21;<br>12.01; |

| | | | | |
|---|---|---|---|---|
| 28 | 227-30°C<br>(AcOH) | ($C_{29}H_{24}N_2O_6S_2$) | 559(M-1)$^-$ | 1.6-2.0; 4.11;<br>4.28; 7.74; 8.38;<br>12.54 |
| 29 | 285-8°C<br>(AcOH) | ($C_{25}H_{24}N_4O_6$)<br>63.02 5.08 11.76<br>63.08 5.00 11.36 | 477(M+1)$^+$ | 1.5-1.83; 4.06;<br>6.40; 6.98; 7.60;<br>10.42; 11.15 |
| 30 | >300°C | ($C_{18}H_{14}N_2O_4S_2$)<br>55.94 3.65 7.25 16.60<br>55.94 3.73 7.14 16.71 | 385(M-1)$^-$ | 3.52; 4.03; 4.99;<br>7.4-8.2; 12.13; |
| 31 | 233-5°C<br>(AcOH) | | 509(M-1)$^-$ | 1.5-1.9; 4.1; 6.76;<br>7.08; 7.80; |
| 32 | 290-5°C<br>(AcOH) | ($C_{24}H_{16}N_2O_6S_2$) | 491(M-1)$^-$ | 4.98; 7.08; 7.48;<br>7.72; 12.51; |

## Claims

**1.** A compound of the general formula

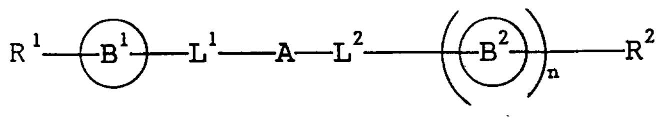

wherein $R^1$ and $R^2$ may be the same or different and each means a group of the formula

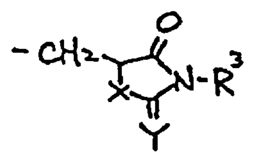 ,

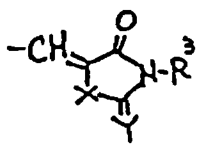 , 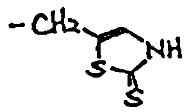 , 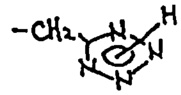 or

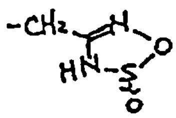 ;

$R^3$ means a hydrogen atom, an acetylamino group, or a lower alkyl group which may be substituted by carboxy or sulfoxy; X and Y are the same or different and each means an oxygen atom, a sulfur atom or a group of the formula NH; $B^1$ and $B^2$ each means a benzene or naphthalene ring which may be substituted by lower alkyl; $L^1$ and $L^2$ are the same or different and each means a single bond, an oxygen atom, a sulfur atom, a sulfinyl group or a sulfonyl group; A means a single bond, a straight-chain or branched alkylene group, a lower $C_2$-$C_6$ alkenylene group or an alkynylene group; n means 0 or 1; provided that where n is equal to 1, $R^1$ and $R^2$ each means

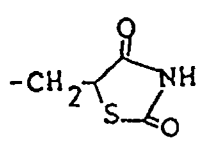 or 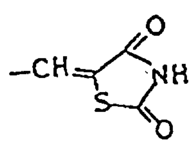 ,

and both of $L^1$ and $L^2$ mean oxygen, a compound wherein A means a straight-chain $C_2$-$C_6$ alkylene group is excluded.

**2.** 4,4'-Bis[(2,4-dioxo-5-thiazolidinyl)methyl]diphenylmethane.

**3.** Cis-1,4-bis[p-[(2,4-dioxo-5-thiazolidinyl)methyl]phenoxy]-2-butene.

**4.** Trans-1,4-bis[p-[(2,4-dioxo-5-thiazolidinyl)methyl]phenoxy]-2-butene.

**5.** A process for producing the compound of claim 1 as set forth in the specification.

# INTERNATIONAL SEARCH REPORT

International Application No PCT/JP91/00887

**I. CLASSIFICATION OF SUBJECT MATTER** (if several classification symbols apply, indicate all) [6]

According to International Patent Classification (IPC) or to both National Classification and IPC

Int. $Cl^5$ C07D233/76, 84, 88, 257/04, 263/44, 46, 48, 277/34, 36, 40, 291/04, A61K31/41, 42, 425

**II. FIELDS SEARCHED**

Minimum Documentation Searched [7]

| Classification System | Classification Symbols |
|---|---|
| IPC | C07D233/76, 84, 88, 257/04, 263/44, 46, 48, 277/34, 36, 40, 291/04, A61K31/41, 42, 425 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | US, A, 4,659,801 (Minesata Mining and Manufacturing Co.), April 21, 1987 (21. 04. 87), Columns 7 to 8 | 1, 2, 3, 4, 5 |
| A | US, A, 4,338,452 (Eli Lilly and Company), July 6, 1982 (06. 07. 82), Page 1 Abstract | 1, 2, 3, 4, 5 |
| A | JP, A, 56-61366 (Dojin Kagaku Kenkyusho K.K.), May 26, 1981 (26. 05. 81), Left column, page 1 (Family: none) | 1, 2, 3, 4, 5 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| September 12, 1991 (12. 09. 91) | October 7, 1991 (07. 10. 91) |
| **International Searching Authority** | **Signature of Authorized Officer** |
| Japanese Patent Office | |